# EUROPEAN PATENT SPECIFICATION

(11) **EP 1 235 536 B1**
(45) Date of publication and mention of the grant of the patent: **25.06.2003**
(21) Application number: 00986137.8
(22) Date of filing: 07.12.2000
(51) Int. Cl.: A61F 2/06, A61K 48/00, A61K 9/00

(54) **MEDICAL DEVICE comprising a synthetic surface having nucleic acid for in vivo induction of its endothelialisation**
MEDIZINISCHE VORRICHTUNG mit nukleinsäurehaltiger synthetischer Oberfläche zur in-vivo Induktion seiner Endothelialisierung
DISPOSITIF MEDICAL avec une surface comprenant un acide nucléique pour induire in-vivo l'endothélialisation

(30) Priority: 07.12.1999 SE 9904454; 23.12.1999 SE 9904747; 31.01.2000 SE 0000285
(43) Date of publication of application: 04.09.2002
(73) Proprietor: Xenerate AB, 751 83 Uppsala (SE)
(72) Inventor: Lahtinen, Mika, 752 37 Uppsala (SE)
(74) Representative: Dahnér, Christer
(86) International application number: SE0002460
(87) International publication number: WO01041674

(56) References cited:
- WO-A1-99/55315
- WO-A2-98/20027
- ERIC VAN BELLE ET AL.: 'Passivation of metallic stents after arterial gene transfer of phVEGF165 inhibits thrombus formation and intimal thickening' J. AM. COLL. CARDIOL. vol. 29, no. 6, May 1997, pages 1371 - 1379, XP002937216

## Description

### Technical field

The invention relates to a medical device suitable for implantation into a human or animal, such as an implantable prosthetic device, as well as a method of producing a device according to the invention.

### Background

Diseased and damaged parts of the body are best repaired or replaced with an organism's own tissue. Physicians and surgeons routinely replace tissue, organs or bone through delicate and complicated medical procedures. Appropriate donor tissues are generally procured elsewhere: either from the recipient's own body (autograft); from a second donor (allograft); or, in some cases, from a donor of another species (xenograft). Tissue transplantation is costly, and suffers from significant failure rates, an increasing risk of disease transmission and inadequate supplies of donor tissues. Therefore, in response to these current transplantation issues, use of artificial or synthetic medical implant devices, fabricated through tissue engineering technology, has been the subject of considerable attention.

Although implant devices can be used in some instances as an alternative to donor-based transplants, they too often produce unsatisfactory results because of the implant's incompatibility with the body and inability to function properly. Lack of normal cell lining of the vascular graft's synthetic surface sets-up conditions that increase the risk of thrombosis, hyperplasia and other medical/surgical procedural complications. Vascular grafts require non-thrombogenic surfaces. Vascular implant materials must have a biocompatible surface, allowing only a minimal response of platelets to the vessel's inner surface; and, at the same time, have the correct fluid dynamics at the vessel wall-blood interface to eliminate or reduce unwanted turbulence and eddy formation. In other types of implants, unwanted fibro-genesis can occur, encasing the implant. The implant will then have an increased risk of dysfunction and other medical complications.

One specific area where implants or grafts are frequently used is in the cardiovascular field. Cardiovascular diseases affect a large segment of the human population, and are a cause for significant morbidity, costs and mortality in the society. About 60 million adults in the USA have a cardiovascular disease, which is the major cause of death in the USA. There are one million acute myocardial infarctions or heart attacks per year with 200000 deaths a year. Claudicatio intermittens causes significant morbidity and yearly 150000 lower limb amputations are required for ischemic disease with significant perioperative mortality. Cerebral vascular disease, strokes and bleedings also causes significant morbidity, costs and mortality. There are one million dialysis patients, and yearly 200000 arteriovenous fistula operations are required to surgically create access for dialysis.

Coronary and peripheral vascular diseases are characterised by blockages in the blood vessels providing blood flow and nutrition to the organs. Other significant disease groups are aneurysms, i.e. local dilatation of the vessels, pseudoaneurysm, and dissection of the vessel wall. There are pharmacological, surgical and percutaneous strategies to treat these diseases. In pharmacological treatment of ischemic heart disease the goal is to make blood less coagulable, and to increase blood flow by vessel dilation or to reduce oxygen consumption.

The surgical treatment for cardiovascular disease is to bypass, substitute or reconstruct a diseased vessel with a vascular graft or patch. Alternatively the vessel can be treated percutaneously or surgically with intraluminal implants, such as adjustable stent structural suppports, tubular grafts or a combination of them. The intent of percutaneous methods is to maintain patency after an occluded vessel has been re-opened, using balloon angioplasty, laser angioplasty, atherectomy, roto-ablation, invasive surgery, thrombolysis, or a combination of these treatments. Stents and tubular grafts can also be used to exclude a local vascular dilatation or dissection.

In coronary artery surgery the obstructed vessel is bypassed with an autologous vascular graft. The operation is called CABG, which means coronary artery bypass grafting. Intracardiac patches are used to repair holes in the cardiac septa or wall. In peripheral artery surgery a graft is usually implanted to bypass an obstruction, for example from the groin to the thigh. In some cases arterial segment may alternatively be replaced with a vascular graft. Prosthetic vascular patches are used in vascular surgery in several operations, which requires an incision in the wall of the blood vessel, such as thrombectomies, endarterectomies, aneurysmal repairs and vessel reconstructions. In percutaneous revascularisation catheters with balloons, stents or stent grafts are used to reduce the narrowing or exclude the dilatation or dissection in different anatomical locations such as cerebral, coronary, renal, other peripheral arteries and veins, and aorta. Balloon dilatations, stents and stent grafts may also be employed in other sites, such as biliary tree, esophagus, bowels, tracheobronchial tree and urinary tract. In access surgery for dialysis there is a need for creating an access to clean the blood with the dialysis machine. Usually a connection called fistula is constructed between the upper extremity artery and vein to create a high blood flow required for dialysis.

More than 350000 vascular grafts are implanted each year and numerous synthetic biomaterials have been developed as vascular substitutes. As a foreign material, grafts are thrombogenic and prone to clot in a higher degree than autologous material. To overcome thrombogenicity, most approaches have concentrated on creating a surface that is thromboresistant, with the majority of these efforts being directed toward an improved polymer surface. Studies have demonstrated that selected materials, for example Dacron and ePTFE (expanded polytetrafluorethylene), successfully can be incorporated in both large and small caliber arteries in animal models (Zdrahala, J Biomater Appl 1996; 10:309-29). In humans, Dacron and ePTFE vascular prostheses have met certain clinical success in large and middle-sized arterial reconstructions, but are yet not ideal. However, the success is limited for vessel substitutes smaller than 6 mm in diameter, due to thrombosis (i.e. propensity to develop clots) and anastomotic hyperplasia (Nojiri, Artif Organs 1995 Jan;19(1):32-8).

In animals, complete endothelialisation of the vascular graft has been shown to occur in 2-4 weeks depending on species. This period without endothelial surface may result in undesired effects and problems due to e.g. thrombogenecity of the surface. In humans, the flow surface remains unhealed except for some case reports (Wu, J Vasc surg 1995 May;21(5):862-7, Guidon, Biomaterials 1993 Jul;14(9):678-93 ), which however, particularly in small vessels, have led to inferior performance compared to autologous grafts (Nojiri, Artif Organs 1995 Jan;19(1):32-8). Berger, Ann of Surg 1972;175 (1):118-27, Sauvage). Autologous grafts, on the other hand, comprises a step for the harvesting thereof, which leads to longer operation times and also possible complications in the harvesting area. Transposition of omentum with uncompromised vasculature around a porous carotid artery PTFE graft has been demonstrated to increase endothelial cell coverage in the graft lumen in dogs (Hazama, J of Surg Res 1999;81;174-180), however, entailing problems with a cumbersome and complex procedure, such as discussed above.

Several strategies have been suggested to improve the patency of synthetic vascular implants. The main strategies have been to modify implant materials or to add chemical compounds to the grafts (e.g. US-A-5,744,515). The substance mostly used has been heparin, which either is bound to the graft, or is given with a local drug delivery device.

Further, grafts have been seeded with endothelial cells, and sodded with endothelial cells or bone marrow (Noishiki, Artif Organs 1998 Jan; 22(1):50-62, Williams & Jarrel, Nat Medicine 1996;2:32-34). In cell seeding, endothelial cells are mixed with blood or plasma after harvesting and then added to the graft surface during the preclotting period. The endothelial cells used in these methods may be derived from either microvascular (fat), macrovascular (for example from harvested veins), or mesothelial sources, whereby the graft later on is implanted. More specifically, these methods comprise several steps, including harvesting of the tissue with endothelial cells, separation of endothelial cells, in some cases a culture of endothelial cells, seeding of endothelial cells on the graft materials and finally implanting the graft. Accordingly, a substantial drawback with these methods is that they are time consuming and cumbersome in practice, and they also require a specific expertise in the area as well as the suitable equipment. Furthermore, such seeded endothelial cells have been genetically engineered, with various results: transduction of the cells with tissue plasminogen activator (tPA) decreases endothelial cell adhesion to the graft surface, and transfection with retrovirus reduces endothelialisation. In order to improve cell seeding, vascular endothelial growth factor (VEGF) transfected endothelial cells or fat cells have been used. In addition to the drawbacks discussed above, this method is even more cumbersome and therefore costly to be useful in practice. A method to transduce endothelial progenitor cells and then re-administer them has been described. However, the problems are still as mentioned above. In order to improve the technology for endothelial cell growth on a surface, ligand treatment of graft surfaces has been suggested. In cell sodding, endothelial cells are administered directly on the polymeric graft surface after harvesting, whereby the graft is implanted, but this technique also includes several steps as mentioned above, which makes it cumbersome as well. Also, tissue engineering, which is also a complex and therefore costly procedure, has been used in order to construct vascular tissues for implantation. Arterial homografts have been described, but they give rise to problems regarding arterial preservation and antigenicity.

Thus, at the moment, there is a great need and interest to improve the endothelialisation and graft healing in clinical practice. However, hitherto, no such methods that works in practice have yet been developed.

Further, in the United States, 500000 stenting procedures are performed in the yearly with in average 1,7 stents per patient. Stents, i.e. relatively simple devices of fine network structures, are well known in the art Stenting for vessel obstruction is usually combined with opening of the artery by dilatation, ablation, atherectomy or laser treatment. Usually, stents are composed of network of some material, usually stainless steel, which is entered to the diseased area usually percutaneously with a catheter. Stents are of different designs for example, self-deployable/ pressure expandable, tubular/ conical/ bifurcated, permanent/temporary, nondegradable/ biodegradable, metal/ polymeric material, with or without antithrombotic medication. They are implanted in a blood vessel in different anatomical locations such as cerebral, coronary, renal, other peripheral arteries and veins, and aorta. Stents may also be used in other locations such as biliary tree, esophagus, bowels, tracheobronchial tree and genitourinary tract. Stents may be used for example to treat stenoses, strictures or aneurysms. Stents characteristically have an open mesh construction, or otherwise are formed with multiple openings to facilitate the radial enlargements and reductions and to allow tissue ingrowth of the device structure. After the vessel dilatation stents have been associated with subacute thrombosis and neointimal thickening leading to obstruction. Before the stent era balloon dilatations alone were used to relieve vessel narrowing. A balloon with hydrogel for delivery of naked DNA have been described (Riessen, Human Gene Therapy 1993, 4:749-758) and also a balloon with hydrogel and gene for drug delivery (US-A- 5,674,192, Sahatjian et al). Catheters have been used to deliver angiogenic peptides, liposomes and viruses with encoding gene to the vascular wall (WO 95/25807, US-A-5,833,651 as above). Catheters have also been used to deliver VEGF protein in order to provide a faster endothelialization of stents (van Belle, Circ. 1997:95 438-448). Further, a hydrogel lined stent for gene delivery (US-A- 5,843,089) and a stent for viral gene delivery (Rajasubramanian, ASAIO J 1994; 40: M584-89, US-A- 5,833,651) have been described. Endothelial cell seeding on the stent has been used as a method to deliver recombinant protein to the vascular wall, in order to overcome thrombosis, but as mentioned above, this technology is cumbersome and therefore costly. In addition, the prior art relating to stents is mainly focused on the prevention of restenosis.

Stent grafts, also referred to as covered stents, are well known in the art. Such stents are a combination of two parts, namely a stent portion and a graft portion. In a stent graft, a compliant graft is coupled to a radially expandable stent. Stent grafts are considered to be usable, by forming a complete barrier between the stent and the blood flow through the vessel. The graft may serve as a biologically compatible inner covering, by preventing turbulent blood flow over the wire members or other structural materials of which the stent is formed, by preventing thrombotic or immunologic reactions to the metal or to other materials of which the stent is made, and by forming a barrier to separate a diseased or damaged segment of the blood vessel from the blood-flow passing there. In humans, the main problem with stent grafts is the lack of complete endothelialisation and formation of neointimal thickening leading to occlusion, as discussed above in relation to grafts. Experimental studies have shown that vascular injuries, that arises when the stent is delivered, induces local expression and release of mitogens and chemotactic factors, which mediates neointimal lesion formation. Stent grafts may be used in aorta, cerebral, coronary, renal, other peripheral arteries and veins, and aorta. Stent grafts may also be used in other locations such as biliary tree, esophagus, bowels, tracheobronchial tree and genitourinary tract.

Yearly, about 100000 heart valve replacement operations are performed. Heart valve prosthesises are well known in the art. There are of four types of grafts: synthetic grafts, xenografts, allografts and autografts. Xenografts are usually preserved pericardial and porcine valves e.g. Carpentier-Edwards, Ionescu-Shiley, Hancock, Pericarbon or stentless valves. Biological degeneration is a major concern in bioprosthetic valves. Degeneration is characterised by disruption of endothelial cell barrier and lack of endothelialisation, increased permeability leading to eased diffusion of circulating host plasma proteins into valve tissue, and increased activity of infiltration processes e.g calcification and lipid accumulation, and biodegradation of the collagen framework. Also a mild to moderate infiltration of inflammatory cells has been described and studies have shown either no (Isomura J Cardiovasc Surg 1986, 27:307-15) or scarce growth of endothelium on bioprosthetic valve surface (Ishihara, Am. J. Card. 1981:48, 443-454) after one year. Several other problems are also associated with valve prosthesis, such as thromboembolism, calcification, infections, hemolysis, perivalvular leaks and anticoagulant related hemorrhage. Bioprosthetic valve endothelialisation could in theory result in prevention of thrombous formation, provide protection against infections, reinforce mechanical strength of the basal regions of the cusps, and present a barrier to the penetration of plasma proteins and other components so decreasing calcific deposits. At the moment there is no tissue valve in the market, which can endothelialise rapidly. Changing the method of preservation, neutralisation of glutaraldehyde preservative and pre-endothelialisation of bioprosthetic valves has been suggested to improve valve performance. Some studies have been made on endothelial seeding in this context, but it is clinically cumbersome due to the many steps required, as described above.

There are several mechanical heart valves, and they usually employ a ball, disc, valve leaflets, or other mechanical devices to regulate the direction of blood flow through the prosthesis. By their nature, mechanical heart valve prosthesises have metal or plastic surfaces when exposed to blood flow. The surfaces are thrombogenic to some degree, due to deficiencies in design, physical structure, operational characteristics and structural material. Leaflets and discs are usually made of pyrolytic carbon, and the orifice ring may be covered by, or made of pyrolytic carbon.

As mentioned above, implantable devices are also used in other fields than the cardiovascular. Various implantable devices have been described, such as for drug delivery, gene therapy, and cell encapsulation purposes. A variety of devices, which protect tissues or cells producing a selected product from the immune system have been explored for implant in a body, such as extravascular diffusion chambers, intravascular diffusion chambers, intravascular ultrafiltration chambers, and microencapsulated cells. However, when foreign biomaterials are implanted, an inflammatory foreign body reaction starts, which in the end encapsulates the device, and inhibits diffusion of nutritive substances to the cells inside the semipermeable membrane. The zone is non-vascular. The lack of vascularity is an obstacle for diffusion of substances. It decreases long term viability of the encapsulated endocrine tissue, and it also makes vascular implants more susceptible to infections. The fibrotic capsule without vascularity can also limit the drug and gene therapy device performance. In US-A-5,882,354, a chamber holding living cells comprises two zones which by an unknown mechanism prevents the invation of connective tissue and increases the close vascularisation of the implant.

Some other materials used in the procedure of implanted devices also encounter similar problems as the ones discussed above. A an example, suture materials can be mentioned, which materials are used for repair, fixation and/or approximation of body tissues during surgical procedures. Strict requirements exist for sutures for attachment of prosthetic devices or implants regarding strength, biocompatibility and biodegradability.

To summarise, the major drawback in this field is that the biocompatibility of the mammalian body, especially the human body, with implanted medical devices cannot be achieved in any satisfactory degree using the prior art methods. In vascular implants, when synthetic materials are used, problems arise due to open thrombotic surfaces where the implant is performed, which in turn generates blood clotting and inferior performance. In synthetic tissue implants, the consequence is a non-vascularised non-nutritive zone, which leads to dysfunction of the implant.

### Summary of the invention

The object of the present invention is to provide a solution to the aforementioned problems. More specifically, one object of the invention is to provide a medical device, which solves the problems of surfaces of medical implants resulting in thrombosis, hyperplasia and other problems. Another object of the present invention is to provide a medical device, which is less cumbersome to use in practice than prior art methods for improving the biocompatibility between foreign materials and the recipient or host thereof. Another object of the invention is to provide a medical device useful in vascular surgery, which entails less risks of being occluded and reoccluded than hitherto known devices. A further object of the invention is to provide a medical device useful as an alternative to homografts but which avoids the risks of antigenicity. Yet another object of the invention is to provide a device useful in measurement and control of metabolic functions which is better accepted and maintained in the human or animal body than prior art devices.

The above given objects and others are according to the present invention achieved by providing a medical device with improved biological properties for an at least partial contact with blood, bodily fluids and/or tissues when introduced in a mammalian body. Said device comprises a core and a nucleic acid present in a biologically compatible medium and is characterised in that said nucleic acid encodes a translation or transcription product capable of promoting endothelialisation *in vivo* at least partially on a synthetic surface of said core.

The nucleic acid is present in the biologically compatible medium in naked form, in a viral vector, such as retrovirus, Sendai virus, adeno associated virus, and adenovirus, or in a liposome.

In one embodiment, the nucleic acid encodes a protein or polypeptide selected from the group consisting of fibroblast growth factor (FGF), platelet derived growth factor (PDGF), transforming growth factor (TGF) and epidermal growth factor (EGF) families, placenta derived growth factor (PIGF), hepatocyte growth factor (HGF) and angiopoetin. Preferably, the nucleic acid encodes vascular endothelial growth factor (VEGF), acidic fibroblast growth factor (aFGF), basic fibroblast growth factor (bFGF) or fibroblast growth factor-5 (FGF-5).

In another embodiment, the biologically compatible medium is a biostable polymer, a bioabsorbable polymer, a biomolecule, a hydrogel polymer or fibrin.

In one advantageoous embodiment, the nucleic acid is present in a reservoir separate from said core enabling a successive delivery thereof to a mammalian body.
In an alternative embodiment, the nucleic acid has been attached to the core by ionic or covalent bonding.

The synthetic surface is either non-porous or porous, in which case it allows capillary and endothelial cell growth through pores. Preferably, the porosity is from about 0 µm to about 2000 µm.

The present device is useful in a wide variety of contexts, and may e.g. be a cardiovascular implant, such as an artificial part of a blood vessel, or an endovascular implant. In general terms, the present device may be used as an implant used for replacement of part of a mammalian body, where said implant is adapted for an at least partial contact with blood, bodily fluids and/or tissues. Further, the present device is useful as a tissue implant or a biosensor. Preferably, the device is selected from the group consisting of vascular grafts, endovascular implants, graft connectors and biosensors.

The present invention also relates to a method of producing a medical device according to the invention.

### BRIEF DESCRIPTION OF THE FIGURES

FIG. 1 shows that transient transfection of HEK293 cells with expression plasmids containing human cDNAs for VEGF165, FGF-2 and FGF-5 results in secretion of the intended proteins.
FIG. 2 shows that human forms of VEGF165, FGF-2 and FGF-5 produced by the expression plasmids stimulate angiogenesis in the chick chorioallantoic membrane assay.
FIG 3. shows that human VEGF mRNA is transcribed after application of the expression plasmid for human VEGF165 to rat abdominal aorta.

### Definitions

Below, explanations are provided as to the meaning of some of the terms used in the present specification. Terms that are not specifically defined herein are to be interpreted by the general understanding thereof within the relevant technical field.

A "medical implant" is here referred to as an implant, a device, scaffold or prosthesis, and is understood as an object that is fabricated for being implanted at least partly in a mammalian. It is intended to be in contact with bodily tissues and fluids providing at least one contacting surface towards the bodily tissues or fluids. A cardiovascular implant is here referred to an implant in a circulatory system, or an implant being connected with the blood-flow, if not specified in any other way. A tissue implant is here referred to as an implant implanted in other bodily tissues or fluids, if not specified in any other way. For example, a medical implant may be an implantable prosthetic device, and more particularly a cardiovascular implant or a tissue implant, as well as a blood-contacting medical implant, a tissue-contacting medical implant, a bodily fluid-contacting medical implant, an implantable medical device, an extracorporeal medical device, an artificial heart, a cardiac assist device, an endoprosthesis medical device, a vascular graft, a stent graft, a heart valve, a cardiovascular patch, a temporary intravascular implant, an annuloplasty ring, a catheter, a pacemaker lead, a biosensor, a chamber for holding living cells, an organ implant, or a bioartificial organ.

An "attached transferable nucleic acid segment" referred to here, represent the wide variety of genetic material, which can be transferred to the tissues surrounding the medical implant. For example, a nucleic acid segment may be a double or single stranded DNA, or it may also be RNA, such as mRNA, tRNA or rRNA, encoding a protein or polypeptide. Optionally the nucleic acid may be an antisense nucleic acid molecule, such as antisense RNA or DNA, which may function by disrupting gene expression. Suitable nucleic acid segments may be in any form, such as naked DNA or RNA, including linear nucleic acid molecules and plasmids, or as a functional insert within the genomes of various recombinant viruses, such as DNA viruses or retroviruses. The nucleic acid segment may also be incorporated in other carriers, such as liposomes or other viral structures. The attached transferable nucleic acid segment is attached to the medical implant in such a way, that it can be delivered to and taken up by the surrounding tissues.

The term "attached" refers to adsorption, such as physisorption, chemisorption, ligand/receptor interaction, covalent bonding, hydrogen bonding, or ionic bonding of the chemical substance or biomolecule, such as a polymeric substance, fibrin or nucleic acid to the implant.

A "surrounding tissue" here refers to any or all cells, which have the capacity to form or contribute to the formation of new endothelial lining or capillarisation of the implant surface. This includes various tissues, such as fat, omentum, pleura, pericardium, peritoneum muscle, vessel wall, and fibrous tissue, but the particular type of surrounding tissue is not important as long as the cells are activated in a way that ultimately gives rise to the endotelialisation or capillarisation of the implant. "Surrounding tissue" is also used to refer to those cells that are located within (excluding cells in tissue chambers), are in contact with, or migrate towards the implant. Also, cells that upon stimulation further attract endothelial cells are considered to be surrounding tissue, as well as cells or tissues that arrive to the active site of cardiovascular implant endothelialisation or tissue implant vascularisation.

An "endothelium" is a single layer of flattened endothelial cells, which are joined edge-to-edge forming a membrane covering the inner surface of blood vessels, heart and lymphatics.

"Endothelialisation" is here referred to the growth of endothelial cells on all mammalian tissue or fluid contacting surfaces of a biomaterial, that is used to form a porous or nonporous implant. Endothelialisation of surfaces can occur via longitudinal growth, ingrowth of capillaries and/or capillary endothelial cells through the pores in the implants, or seeding of circulating endothelial precursor cells. In this disclosure, it will be used interchangeably with the phrase "capillary endothelialisation", to refer to the growth of endothelial cells on substantially all tissue contacting surfaces of a biomaterial, that is used to form a porous or nonporous implant, unless otherwise specified.

The terms "capillarisation" and "vascularisation" are here understood as the formation of capillaries and microcirculation on the implant surface, and they will be used interchangeably with endothelialisation, unless otherwise specified.

"Angiogenesis" and reflections thereof, such as "angiogenic", are here referred to formation and growth of endothelial cells in the existing mammalian tissue, such as in the surrounding tissue.

A translational or a transcriptional product having "the potential to promote endothelialisation" of the medical implant, is here understood as a chemical substance or biomolecule, preferably a hormone, a receptor or a protein, more preferably a growth factor, which, as a result of its activity, can induce endothelialisation or capillarisation of the medical implant.

"Porosity" and reflections thereof, such as "pores" and "porous", are here referred, if not otherwise specified, to a biomaterial having small channels or passages, which start at a first surface and extend substantially through to a second surface of the biomaterial.

"Surface" refers to the interface between the biomaterial and its environment. It is intended to include the use of the word in both its macroscopic sense (e.g. two major faces of a sheet of biomaterial), as well as in its microscopic sense (e.g. lining of pores traversing the material).

The term compartment refers to any suitable compartment, such as for example a vial or a package.

The references having seven digits (e.g. 4,654,321), that are used throughout this specification, refers to numbers of US patent applications, if nothing else is specified.

### Detailed description of the invention

In a first aspect, the present invention relates to a medical device with improved biological properties for an at least partial contact with blood, bodily fluids and/or tissues when introduced in a mammalian body, which device comprises a core and a nucleic acid present in a biologically compatible medium, characterised in that said nucleic acid encodes a translation or transcription product capable of promoting endothelialisation *in vivo* at least partially on a synthetic surface of said core. The nucleic acid is provided in a way whereby transfer thereof into cells of tissue surrounding the implant is allowed. In the present specification, it is to be understood that the term "introduced in a mammalian body" is used in a broad sense to encompass both devices that are totally included in a body and devices which are only in part introduced, but wherein at least one surface made from a synthetic material is in contact with blood, bodily fluids and/or tissues of said body.

The endothelium formed according to the invention on the synthetic surface offers many of the advantages of a native surface. Endothelium is a single layer of flattened cells, which are joined edge to edge forming a membrane of cells covering the inner surface of blood vessels, heart and lymphatics. In theory, endothelialisation of the graft can occur either via longitudinal growth from the anastomosis area (transanastomotic), ingrowth of capillaries and/or capillary endothelial cells through the synthetic surface, such as a graft wall, and into porosities (transinterstitial), or seeding of circulating endothelial precursor cells. In the transinterstitial migration through the pores, the endothelial cells originate from capillaries through attachment, spreading, inward migration and proliferation.

Thus, even though efforts have been made in the prior art to avoid thrombogenecity and the resulting clotting on polymeric surfaces of vascular grafts, such efforts have not proved satisfactory with smaller vessels, wherein thrombosis and hyperplasia have caused substantial problems. The present invention provides for the first time a device comprising at least one synthetic surface, which is capable of being accepted by the body due to the formation of an endothelial layer thereon. The present invention provides a versatile technology useful with a large range of implants, and surprisingly also efficient with small size synthetic vessel sections that have previously been known to clot. The endothelial layers formed according to the invention have not been observed to form in humans according to the prior art, and formation thereof in animals have been observed, but due to a very slow growth, not in any extent sufficient to avoid the problems associated therewith, as shown in the examples below.

In one embodiment of the device according to the invention, the nucleic acid is present in the biologically compatible medium in naked form. Riessen (JACC 1994:5, 1234-1244) has delivered naked DNA to a prior art stent as a balloon with hydrogel for delivery of naked DNA. However, in that case, the purpose of said DNA was to prevent restenosis in the network of the stent, contrary to the delivery according to the present invention, whereby a novel endothelial layer is created on a surface. In an alternative embodiment, the nucleic acid has been introduced in a viral vector selected from the group consisting of retrovirus, Sendai virus, adeno associated virus and adenovirus. In yet another embodiment, the nucleic acid is present in a liposome.

The use of gene transfer has been postulated for the treatment or prevention of diseases in several publications. Gene therapy entails the use of genetic material as the pharmacological agent. While originally recognised as a means for treating hereditary diseases, gene therapy is now understood as a powerful tool for delivering therapeutic mRNA or proteins for local and/or systemic use. There are two approaches in gene therapy: *ex vivo* and *in vivo*. In the *ex vivo* approach, cells removed from the host are genetically modified *in vitro* before they are returned to the host, and in the *in vivo* approach the genetic information itself is transferred directly to the host without employing any cells as a vehicle for transfer. The gene can be targeted depending on where they are needed, either in stem cells or *in situ*. The principle for gene therapy is that the cell functions are regulated through the alteration of the transcription of genes and the production of a gene. transcription product, such as a polynucleotide or a polypeptide. The polynucleotide or the polypeptide then interacts with other cells to regulate the function of that cell. This transcription change is accomplished with gene transfer. Losordo et al (Circulation 1994, 89:785-792) have shown that gene products that are secreted may have profound biological effects even when the number of transduced cells remains low in contrast to genes that do not encode a secretory signal. For genes expressing an intracellular gene product a much larger cell population might be required for that intracellular gene product to express its biological effects and subsequently more efficient transfection may be required (Isner et al, Circulation, 1995, 91:2687-2692). To illustrate the use of gene therapy this far, genes have e.g. been transferred to adipocytes having a particular utility with respect to diseases or conditions that can be treated directly by *in vivo* gene gene transfer to adipocytes. Transfer of nucleic acids into bone tissue has been shown *in situ* and the use of infected mesothelium either *in situ* or after isolation as therapeutic resource has also been described.

An extremely wide variety of genetic materials can be transferred to the surrounding tissues using the compositions and methods of invention. For example, the nucleic acid may be DNA (double or single stranded) or RNA (e.g. mRNA, tRNA, rRNA). It may also be a coding nucleic acid, i.e. one that encodes a protein or a polypeptide, or it may be an anti-sense nucleic acid molecule, such as anti-sense RNA or DNA, that may function to disrupt gene expression. Alternatively, it may be an artificial chromosome. Thus, the nucleic acids may be genomic sequences, including exons or introns alone, or exons and introns, or coding DNA regions, or any construct that one desires to transfer to the tissue surrounding the prosthesis to promote endothelialisation. Suitable nucleic acids may also be virtually any form, such as naked DNA or RNA, including linear nucleic acid molecules and plasmids, or a functional insert within the genomes of various recombinant viruses, including viruses with DNA genomes, and retroviruses. The nucleic acid may also be incorporated in other carriers, such as liposomes and other viral structures.

Chemical, physical, and viral mediated mechanisms are used for gene transfer. Several different vehicles are employed in gene transfer. There are a number of viruses, live or inactive, including recombinant viruses, that can be used to deliver a nucleic acid to the tissues, such as retroviruses, lentivirus, adenoviruses (e.g. 5,882,887, 5,880,102) and hemagglutinating viruses of Japan (HVJ or Sendai virus) (5,833,651). Retroviruses have several drawbacks *in vivo* which limit their usefulness. They provide a stable gene transfer, but current retroviruses are unable to transduce nonreplicating cells. The potential hazards of transgene incorporation into the host DNA are not warranted if short-term gene transfer is sufficient. Replication deficit adenoviruses are highly efficient and are used in a wide variety of applications. The adenovirus enters the cell easily through receptor interactions, which has been used as a means for transporting macromolecules into the cell. Non-viral nucleic acids can be packaged within the adenovirus, either as a substitute for, or in addition to normal adenoviral components. Non-viral nucleic acids can also be either linked to the surface of the adenovirus or in a bystander process co-internalised and taken along as a cargo in the receptor-endosome complex. Adenovirus-based gene transfer does not result in integration of the transgene into the host genome, and is therefore not stable. It also transfects nonreplicable cells. The limited duration of angiogenic protein expression is sufficient for angiogenesis, transient gene transfer for endothelialisation, and healing of the vascular prosthesis in coronary and peripheral locations. Other examples of used viral vectors are adenoassociated viruses (AAV), herpes viruses, vaccinia viruses, lentivirus, poliovirus, other RNA viruses and influenza virus (Mulligan, Science 1993; 260: 926-32; Rowland, Ann Thorac Surgery 1995, 60:721-728). DNA can also be coupled to other types of ligands promoting its uptake and inhibiting its degradation (e.g. 5,972,900, 5,166,320, 5,354,844, 5,844,107, 5,972,707). It can also be coupled to a so called cre-lox system (Sauer&Henderson, Proc Natl Acad Sci; 1988, 85:5166). Naked DNA can also be given and the empirical experience is consistent with that double stranded DNA is minimally immunogenic and is unlikely elicit an immunologic reaction. Naked DNA encoding for VEGF has been shown to increase angiogenesis when given in an ischemic hind limb model (Pu et al, J Invest Surg, 1994;7:49-60). Also other growth factors have been effective in the same model (Ferrara & Alitalo, 1999;12:1359-64). Naked DNA encoding for VEGF has also been used clinically in ischemic peripheral vascular disease (Isner et al, Lancet, 1996;348:370-4, Baumgartner et al, Circulation, 1998;97:1114-23) and currently at least two trials are ongoing for ischemic heart disease with naked DNA and an adenovirus carried gene encoding for VEGF. The results will be published during the spring 2000. (Hughes SCRIP 1999 Nov 2493:24). Adenovirus carried gene encoding for FGF-5 has also been used for intramyocardial injections (5,792,453).

Liposome-DNA complex has a lower efficacy than adenoviral transfection. Transfection efficacy is improved when cells are proliferating. The traditional chemical gene transfer methods are calcium phosphate co-precipitation, DEAE-dextran, polymers (5,972,707), and liposome-mediated transfer (for example 5,855,910, 5,830,430, 5,770,220), and the traditional physical methods are microinjection, electroporation (5,304,120), iontophoresis, a combination of iontophoresis and electroporation (5,968,006), and pressure (5,922,687) (Rowland). Transfection efficiency can also be improved by pharmacological measures i.e. addition of PEI.

The invention may be employed to promote expression of a desired gene in tissues surrounding an implant, and to impart a certain phenotype, and thereby promote prosthesis endothelialisation or vascularisation. This expression could be increased expression of a gene that is normally expressed (i.e. over-expression), or it could be the expression of a gene that is not normally associated with tissues surrounding the prosthesis in their natural environment. Alternatively, the invention may be used to suppress the expression of a gene that is naturally expressed in such tissues, and again, to change or alter phenotype. Gene suppression may be a way of expressing a gene that encodes a protein that exerts a down-regulatory function. It may also utilise anti-sense technology.

Thus, the nucleic acids used with the device according to the present invention encode transcription or translation products capable of promoting or stimulating endothelialisation *in vivo*, i.e. they are angiogenic factors. Thus, in one embodiment, the nucleic acid encodes a protein or polypeptide selected from the group consisting of fibroblast growth factor (FGF), platelet derived growth factor (PDGF), transforming growth factor (TGF) and epidermal growth factor (EGF) families, placenta derived growth factor (PlGF), hepatocyte growth factor (HGF), and angiopoetin. In one specific embodiment, the nucleic acid encodes vascular endothelial growth factor (VEGF), acidic fibroblast growth factor (aFGF), basic fibroblast growth factor (bFGF) or fibroblast growth factor-5 (FGF-5).

WO 98/20027 has described the therapeutic use of an agent that stimulates NO or prostacyclin production in the treatment of intimal hyperplasia, hypertension and atherosclerosis. More specifically, such an agent, e.g. vascular endothelial growth factor (VEGF), is delivered to the exterior of a blood vessel using a delivery reservoir in the form of a collar placed around the vessel. The collar then directs said agent to the vessel while diffusion thereof into the surrounding tissue is avoided. Even though the WO 98/20027 delivery device is similar to the present invention as regards the components used, the nature thereof is distinctively different. The present invention provides a nucleic acid encoding an angiogenic factor, such as a VEGF gene, to tissue that surrounds a synthetic device, which is introduced into the body e.g. to replace or support a native organ. Said nucleic acid enters cells of said tissue and provides the expression of one or more substances that stimulates the endothelial growth of the synthetic surface. The purpose of WO 98/20027 is quite the contrary, since the aim thereof is to deliver a gene, such as VEGF, to a specific site in the body, where said gene will be expressed and provides an effect that in fact is capable of suppressing any cell growth at the delivery site, i.e. it prevents hyperplasia. These totally contrary effects are achieved since the mode of delivery is different; the present invention provides a wide spread delivery of the nucleic acid to the surrounding tissue in order to obtain as high expression as possible, while the WO 98/20027 delivery is designed to provide a directed administration to a specific site. Said directed administration is according to WO 98/20027 obtained by using a synthetic device in the form of a collar, which limits the dispersion of gene at the site of delivery, while no such limitation is used in the present invention. Thus, even though WO 98/20027 also uses a synthetic device, contrary to the present invention, no endothelial layer is created thereon. In fact, should a novel endothelial layer be created on the synthetic WO 98/20027 collar, that in itself would be a sign of failure of the intended purpose, which clearly illustrates the differences between WO 98/20027 and the invention.

Direct administration of angiogenic proteins or peptides to obtain new vessel development has been described in scientific reports. The several members of the fibroblast growth factor (FGF) family: a-FGF, b-FGF, FGF-4, FGF-5, TGF-family, EGF-family, PDGF-family, such as any VEGF-family isomer, angiopoetin (Ang) family, such as Ang1/Ang2 and others like PIGF, have been implicated in the regulation of angiogenesis (for example, Ferrara et al, J Cellular Biochem, 1991, 47:211-218, Folkman et al, J Biol Chem 1992;267:10931-34, Klasbrun et al, Ann Rev Physiol, 1991;53:217-39, Harada et al J Clin Invest 1994;94:623-30, Yanagisawa-Miwa et al, Science 1992;257:1401-03, Baffour et al, J Vasc Surg 1992;16;181-191, Takeshita et al, J Clin Invest 1994;93:662-670, Shing et al, Science,1984:223:1296-99, Korpelainen & Alitalo, Curr Opin Cell Biol, 1998;10:159-64, Ferrara & Alitalo, Nat Med, 1999;12:1359-63, 5,928,939, 5,932,540, 5,607,918). However, a prerequisite for achieving an angiogenic effect with these proteins has been the need for repeated or long term delivery of the protein, which limits the utility of using these proteins to stimulate endothelial growth in clinical setting. Some of VEGF isomers are heparin binding angiogenic growth factors, which can be secreted from intact cells because of a signal sequence. Also, FGF-5 is synthesised and secreted from the transfected cells to the interstitium where it induces angiogenesis (5,792,453). VEGF is specific in its mitogenic effects to endothelial cells because its high affinity receptors are present on endothelium. Among the other growth factors FGF-1 together with mixture of fibrin glue and heparin has been shown to increase transmural endothelialisation through 60 microns internodal distance ePTFE grafts (Gray et al, J Surg Res 1994 Nov, 57(5):596-612). VEGF protein in combination with heparin and biological glue has been described to *ex vivo* specifically to stimulate endothelial cell proliferation. (Weatherford et al, Surgery, 1996, 120: 439). VEGF protein also promotes transgraft endothelial cell growth when combined with bFGF, gelatin and heparin (Masuda, ASAIO J 1997, 43; M530-534). FGF protein is described to have similar effects than VEGF when used together with heparin. (Doi et al, J Biomed Mat Res 1997, 34:361-370). Clinically, both FGF and VEGF protein injections in myocardium have been used to induce angiogenesis in patients with coronary artery disease. bFGF and aFGF protein have also been shown to increase valve endothelialisation *in vitro* and in subcutaneous tissue (Fischlein et al, Int J Artif Organs 1994 Jun;17(6):345-352, Fischlein et al, J Heart valve Dis 1996 Jan;5 (1):58-65) VEGF study has been discontinued and the final results of the FGF study will be published during the spring 2000 (Hughes, SCRIP 1999 Nov; 2493:24). Further, WO 91/02058 has described the administration of a hybrid protein to this end. In summary, all of these reports of use of protein or peptides entails a cumbersome and costly procedure, since the administered protein will only be capable of exerting its function once and then disappear by transport, degradation etc. Contrary to this, the present invention enables a more prolonged delivery, which advantageously can be controlled by engineering the vector, than what was possible by the direct administration of protein.

In another embodiment, the biologically compatible medium is a biostable polymer, a bioabsorbable polymer, a biomolecule, a hydrogel polymer or fibrin. In a specific embodiment, the medium is a mucin composition.

The synthetic surface of the device according to the invention may be either non-porous or porous. Thus, porous, as well as nonporous, implant materials may be used to produce the device, depending on the implant embodiment. For example, graft porosity has been shown to be of importance in vascular graft endothelialisation in animals (Wesolowski, Thorac Cardiovasc Surgeon 1982;30:196-208, Hara, Am J Surg;1967;113:766-69). Further, in the context of mechanical heart valves, porous surfaces have been shown to increase tissue growth and endothelialisation of the valve rings (Bjork, Scand J Thorac Cardiovasc Surg 1990; 24 (2):97-100). In the context of sutures, porous sutures have been described to promote tissue ingrowth into the sutures or promote endothelialisation of the sutures (4,905,367, 4,355,426). In porous grafts, such as vascular grafts, capillary and endothelial cell growth is allowed through pores, and the porosity thereof may be from 0 µm to 2000 µm.

In one embodiment, the nucleic acid has been attached to the core by ionic or covalent bonding.

In one advantageous embodiment, the nucleic acid is present in a reservoir separate from said core enabling a successive delivery thereof to a mammalian body. The tissue surrounding an implanted device can e.g. be pleura, pericardium, peritoneum, fascia, tendon, fat, omentum, fibrous, muscle, skin, or any other tissue in which angiogenesis is required.

Genes expressing angiogenic factor are then attached to the implant or administered in the tissue surrounding the device. The cells in the surrounding tissue become transfected and stimulate angiogenesis and result in endothelialisation and/or capillarisation of the implant, a process that results in endothelialised or vascularised surface with the earlier described advantages of such a surface.

The surface of the present device may be treated in a variety of ways, in all or parts thereof, e.g. by coating, adding fibrin glue or adhesion molecules, as is discussed in more detail below in the experimental section in the general disclosure of materials and methods. The optimal internodal distance for PTFE grafts has been approximately 60 um.

The present device is useful in a wide variety of contexts and depending on the intended use, it may be made from a biomaterial selected from the group of non-soluble synthetic polymers, metals and ceramics with or without modification of the prosthesis surfaces.

Thus, in one embodiment, the device is an implant made of a biocompatible material selected from the group consisting of metal, titanium, titanium alloys, tin-nickel alloys, shape memory alloys, aluminium oxide, platinum, platinum alloys, stainless steel, MP35N, elgiloy, stellite, pyrolytic carbon, silver carbon, glassy carbon, polymer, polyamide, polycarbonate, polyether, polyester, polyolefin, polyethylene, polypropylene, polystyrene, polyurethane, polyvinyl chloride, polyvinylpyrrolidone, silicone elastomer, fluoropolymer, polyacrylate, polyisoprene, polytetrafluoretylene, rubber, ceramic, hydroxyapatite, human protein, human tissue, animal protein, animal tissue, bone, skin, laminin, elastin, fibrin, wood, cellulose, compressed carbon and glass.

Thus, the device may be a medical implant selected from the group consisting of a blood-contacting medical implant, a tissue-contacting medical implant, a bodily fluid-contacting medical implant, an implantable medical device, an extracorporeal medical device, an endoprosthesis medical device, a vascular graft, an endovascular implant, a pacemaker lead, a heart valve, temporary, intravascular implant, a catheter, pacemaker lead, biosensor or artificial organ. In one specific embodiment, the device is a cardiovascular implant, such as an artificial part of a blood vessel, or an endovascular implant. In general terms, the present device may be used as an implant used for replacement of a part of a mammalian body, where said implant is adapted for an at least partial contact with blood, bodily fluids and/or tissues. Further, the present device is useful as a tissue implant or a biosensor. In alternative embodiments, the present device may be any other bioartificial implant that provides a metabolic function to a host, such as a pump for the delivery of insulin etc.

In fact, the present device may be virtually any one of a variety of devices, which protect tissues or cells producing a selected product from the immune system have been explored for implant in a body, such as extravascular diffusion chambers, intravascular diffusion chambers, intravascular ultrafiltration chambers, and microencapsulated cells. Cells can be derived from other species (xenografts), they can be from the same species but different individuals (allografts), and sometimes they are previously isolated from the same individual but are modified (autografts). Bioartificial implants are designed to provide a needed metabolic function to a host, either by delivering biologically active moieties, such as insulin in diabetes mellitus, or removing harmful substances. Membranes can be hydrophobic, such as PTFE and polypropylene, or hydrophilic, such as PAN/PVC and cuprophane.

More specifically, implants encompassed by the invention include, but are not limited to, cardiovascular devices, such as artificial vascular prosthesises, cardiovascular patches, stent grafts, prosthetic valves, artificial hearts, cardiac assist devices, anastomotic devices, graft connectors, annuloplasty ring, indwelling vascular catheters, pacemaker wires, anti-embolism filters, stents and stent grafts for other indications, and tissue implants, such as chambers holding living cells for implantation, biosensors, surgical suture materials, surgical nets, pledgets and patches, tracheal cannulas, bioartificial organs, surgical implants, plastic surgical implants and orthopedic implants. It is anticipated that the herein described procedures may lead to the development of other artificial organs or devices.

In a second aspect, the invention provides a method for producing an implantable medical device. The device can be formed either by the pretreating of a biomaterial with genes, and then fabricating the device from the treated biomaterial, or by first fabricating the device and then treating the exposed surfaces of the device.

In a third aspect, in general terms, methods for endothelialisation or capillarisation of medical implants by transferring a nucleic acid to the surrounding tissues are described. The methods generally comprise to contact the tissue, surrounding the vascular or tissue implant, with a composition comprising a nucleic acid, in a manner effective to transfer said nucleic acid into the tissue, and to promote endothelialisation of the vascular grafts, cardiovascular patches, stent grafts, heart valves, indwelling vascular catheters, cardiac assist devices and artificial hearts, or to promote vascularisation of tissue implant surfaces. The tissue may be wrapped around the vascular- or tissue implant - nucleic acid composition before implantation to the body. Alternatively, the nucleic acid sequence-prosthesis composition may be implanted in the tissues, or the nucleic acid may be applied to the implantation site before or after the prosthesis implantation, in order to effect, or promote, nuclear acid transfer into the surrounding tissues *in vivo*. In the transferring of nucleic acids into surrounding tissues, the preferred method involves to first add the genetic material to the tissue compatible medium, to impregnate the prosthesis with the nucleic acid-medium composition, and then to use the impregnated prosthesis to contact an appropriate tissue site. Alternatively, the tissue compatible medium can first be administered on the implant, then the nucleic acid is added, whereafter the nucleic acid-prosthesis composition is applied to the implantation site. Alternatively nucleic acid is administered to the tissues surrounding the implant, whereafter the implant is implanted, or the implant is first implanted, whereafter the nucleic acid is administered on the implant. Also, an impregnated implant can be used in combination with administration of nucleic acid in the tissues surrounding the implant before or after implantation. When surrounding tissue is scarce and have a low amount of endothelial cells, the impregnated prosthesis can be surgically wrapped in a tissue of higher endothelial cell content before implantation. Some of the cardiovascular implants, such as vascular prosthesis, cardiovascular patch and stent grafts, have a porosity that is high enough to allow growth of endothelial cells through the pores, and some other cardiovascular implants, such as heart valves are non-porous.

More specifically, the method for endothelialisation of medical implants by transferring a nucleic acid to the surrounding tissues may be disclosed as a method of improving a mammalian, e.g. a human, body's acceptance of a synthetic surface, which method comprises introducing a device comprising a synthetic surface in the body with an at least partial contact with blood, bodily fluids and/or tissues and administering a nucleic acid present in a biologically compatible medium to the surroundings thereof. The method is characterised in that the nucleic acid encodes a translation or transcription product capable of promoting endothelialisation *in vivo* at least partially on said synthetic surface, said administration of nucleic acid being performed before, simultaneously as or after the introduction of the device in the body. As discussed above in relation to the device according to the invention, the nucleic acid can e.g. be administered in naked form, in a viral vector such as a retrovirus, a Sendai virus, an adeno associated virus or an adenovirus, or in a liposome.

Depending on the nature of the device, i.e., the condition of the patient who is to receive the implant, the nucleic acid may encode a protein or a polypeptide selected from the group consisting of fibroblast growth factor (FGF), platelet derived growth factor (PDGF), transforming growth factor (TGF) and epidermal growth factor (EGF) families, placenta derived growth factor (PlGF), hepatocyte growth factor (HGF) and angiopoetin, and specifically vascular endothelial growth factor (VEGF), acidic fibroblast growth factor (aFGF), basic fibroblast growth factor (bFGF) or fibroblast growth factor-5 (FGF-5).

In one embodiment, the nucleic acid is administered to the surroundings of the device, i.e. the tissue, before introduction thereof in a mammalian body. Alternatively, the nucleic acid is administered to such surroundings after the introduction thereof. As the skilled in this field will realise, combinations of such administrations are possible, such as a first administration of a certain amount to the surroundings, the introduction of the device, and thereafter one or more additional administration, either according to a predetermined scheme or depending on the body's acceptance thereof and the rate of growth of the new endothelial layer on the synthetic surface.

In another embodiment, the nucleic acid is administered or attached to the device before introduction thereof in a mammalian body. In a specific ebodiment, this is achieved by attaching the nucleic acid to the core by ionic or covalent bonding. This embodiment may if appropriate be combined with the last mentioned above, so as to provide a method wherein the device has been pretreated with nucleic acid, while the tissue surrounding the device is later supplemented with further additions of nucleic acid present in a suitable carrier. In one embodiment which is advantageous due to its simplicity, said carrier is sterile water or a sterile aqueous solution.

In alternative embodiments of the present method, the biologically compatible medium is a biostable polymer, a bioabsorbable polymer, a biomolecule, a hydrogel polymer or fibrin.

The present method may be used in the context of any mammalian, such as in the treatment of humans to increase the biocompatibility of a foreign, at least partly synthetic, device, such as a medical implant. Further, the present method may be used in monitoring, where a biosensor or other similar equipment is introduced.

Thus, as mentioned above and as further detailed below, the device used in the present method may be an implant used in cardiovascular surgery, a device replacing a part of the body, such as a vessel, a device for introduction into a human body, such as an endovascular implant, a tissue implant, or a biosensor.

In summary, with respect to the transfer and expression of therapeutic genes according to the present invention, the ordinary skilled artisan is aware that different genetic signals and processing events control levels of nucleic acids and proteins/peptides in a cell, such as transcription, mRNA translation, and post-translational processing. These steps are affected by various other components also present in the cells, such as other proteins, ribonucleotide concentrations and the like.

Accordingly, in general terms, the present invention concerns angiogenic devices, which devices may be generally considered as molded or designed vascular implant-gene compositions. The devices of the invention are naturally a tissue-compatible implant in which one or more angiogenic genes are associated with the implant. The combination of gene(s) and implant components is decided by the skilled in this field in order to render the device capable of stimulating angiogenesis when implanted. Devices according to the invention may be of virtually any size or shape, so that their dimensions are adapted to fit the implantation site in the body.

### DETAILED DESCRIPTION OF DRAWINGS

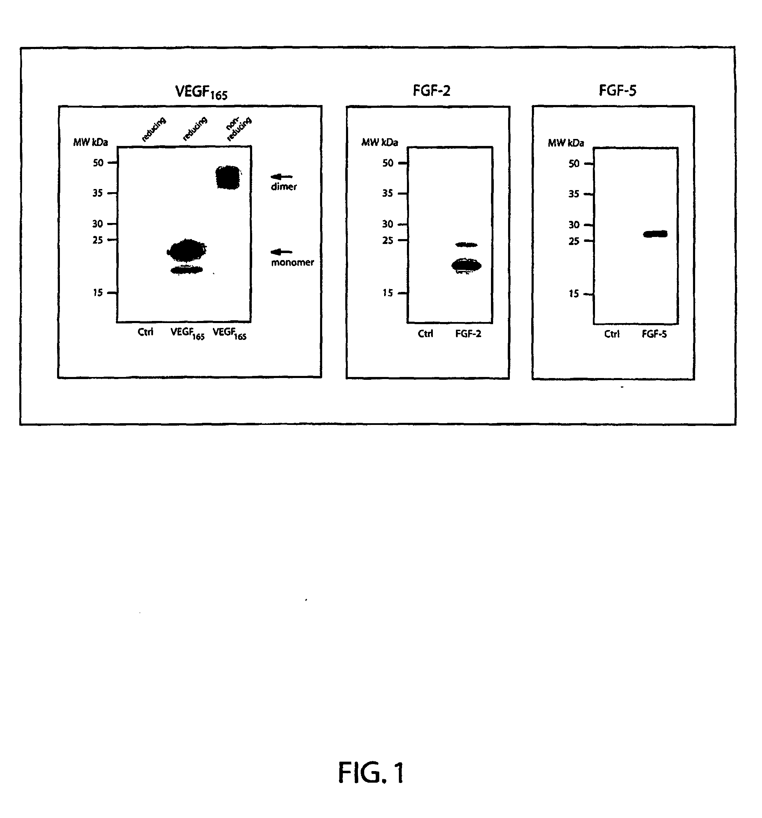

FIG. 1 shows a western blot analysis of secreted human VEGF165, FGF-2 and FGF-5. The expression plasmids pNGVL1-β-gal (negative control), pNGVL3-VEGF165, pNGVL7-FGF-2 and pNGVL3-FGF5 were separately transiently transfected into HEK293 cells using the calcium-phosphate technique. Cells were rinsed with PBS 24 h after transfection and serum free media added to the cells. This media was collected after an additional 24 hours of incubation and analyzed for VEGF165, FGF-2 and FGF-5 proteins by western blotting with specific antibodies. VEGF165 dimerized under non-reducing conditions as expected.

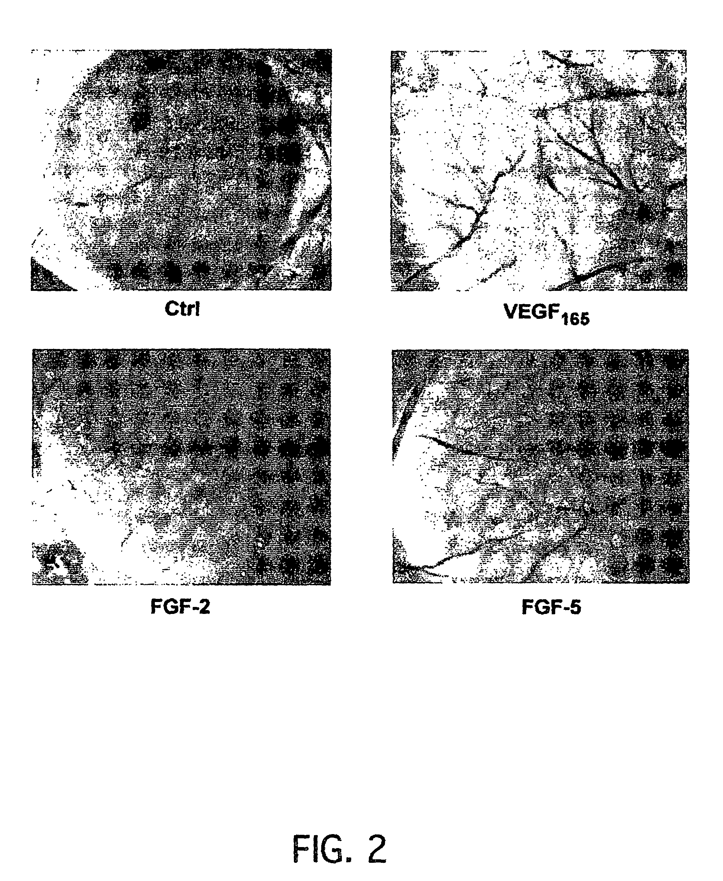

FIG. 2 shows that conditioned media containing VEGF165, FGF-2 or FGF-5, from transiently transfected HEK293 cells, stimulate angiogenesis in the chick chorioallantoic membrane assay. Conditioned media from HEK 293 cells transiently transfected with the control plasmid pNGVL1-β-gal had no stimulatory effect. Conditioned media (10 µl) was applied to a filter disc which was then placed on an avascular zone of the chorioallantoic membrane. Filters were cut out and photographed 3 days later.

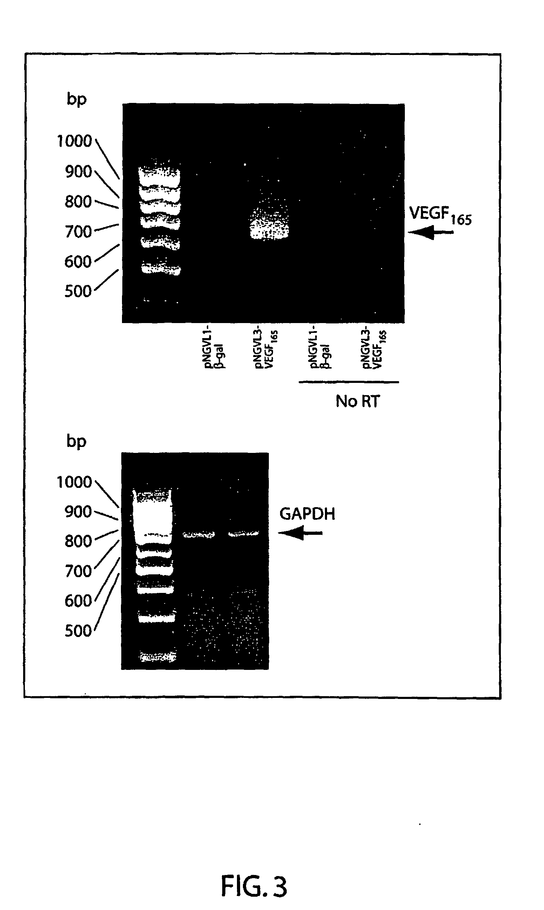

FIG. 3 shows that application of the expression plasmid pNGVL3-VEGF165 produces mRNA when applied to the rat abdominal aorta *in vivo*. 600 µg of pNGVL3-VEGF165 was added around the abdominal aorta of the rat. Abdominal aorta and surrounding tissue was cut out 7 days later and immediately frozen in liquid nitrogen. Total RNA was extracted and reverse transcribed using oligo dT primers. PCR with a sense primer based on vector sequence immediately upstream of the human VEGF165 cDNA insert and an antisense primer based on the human VEGF165 sequence resulted in amplification of the expected fragment. No amplified product could be detected if reverse transcriptase (RT) was omitted. PCR of cDNA from tissues transfected with the control plasmid pNGVLl-β-gal did not result in any amplified product. Primers for a part of GAPDH were used to show that the prepared cDNAs were of good quality.

### EXPERIMENTAL

The following section is provided to illustrate the present invention and should not be interpreted as limiting the invention in any way.

The present experimental section will first describe alternative materials and methods that may be utilised in this context in order to offer as many possibilities as possible within the scope of the appended claims. Thereafter, under the headline examples, specific disclosures of the experiment carried out to describe the effect of the invention and the advantages thereof will be provided.

### Materials and methods

### 1. The nucleic acids

### Implant endothelialisation promoting genes:

As used herein, the term "implant endothelialisation promoting gene" is used to refer to a gene or a DNA coding region that encodes a protein, a polypeptide or a peptide, that is capable of promoting, or assisting in promotion of implant endothelialisation or vascularisation, or that increases the rate of the implant endothelialisation or vascularisation. The terms promoting, inducing and stimulating are used interchangably throughout this text, to refer to direct or indirect processes that ultimately result in the formation of implant endothelium and/or capillaries, or in an increased rate of implant endothelialisation and/or capillarisation. Thus, an implant endothelialisation promoting gene is a gene, which, when it is expressed, causes the phenotype of the cell to change, so that the cell either differentiates, stimulates other cells to differentiate, attracts implant endothelialisation promoting cells, or otherwise functions in a manner that ultimately gives rise to new implant endothelium.

In general terms, a vascular implant endothelialisation promoting gene may also be characterised as a gene capable of stimulating the growth of endothelium in the tissues surrounding vascular prosthesis and thereby promoting the endothelialisation or the vascularisation of the implant. Thus, in certain embodiments the methods and compositions of the invention may be to stimulate growth of endothelium in vascular prosthesis itself and also in tissues surrounding it.

A variety of angiogenetic hormones are now known, of which all are suitable for use in connection with the present invention. Angiogenic genes and proteins that they code for include, for example, hormones, many different growth factors and cytokines, growth factor receptor genes, enzymes and polypeptides. Examples of suitable angiogenetic factors include those of the PDGF super-family, such as VEGF in all variants, fibroblast growth factors, such as acidic FGF, basic FGF and FGF-5, TGF-gene family, including TGFs 1-4, and TGF-beta, angiopoetin-family, such as Ang1 and Ang2, and tumour necrosis factors a-TNF, b-TNF, and PIGF and HGF/SF.

Certain preferred angiogenic genes and DNA are VEGF and those of the FGF family. There is a considerable variation in the terminology currently employed in the literature referring to genes and polypeptides. It will be understood by those skilled in the art, that all genes that encode an active angiogenic protein are considered for use in this invention, regardless of the differing terminology that may be employed. For example, VEGF may be referred to as vascular permeability factor or vasculotropin and bFGF may be referred to as FGF-2.

The DNA sequences for several angiogenic genes have been described both in scientific articles and in U.S. patents, such as 5,928,939, 5,932,540, 5,607,918, 5,168,051, 4,886,747 and 4,742,003.

As disclosed in the above patents, and known to those skilled in the art, the original source of a recombination gene or a DNA to be used in a therapeutic regimen need not be of the same species as the animal to be treated. In this regard, it is contemplated that any recombinant angiogenic gene may be employed to promote vascular prosthesis endothelialisation in a human subject or an animal, such as e.g, horse. Particularly preferred genes are those from human, as such genes are most preferred for use in human treatment regiments. Recombinant proteins and polypeptides encoded by isolated DNA and genes are often referred to with the prefix r for recombinant and rh for recombinant human.

To prepare an angiogenic gene, gene segment or cDNA, one may follow the teachings disclosed herein and also teachings of any of the patents or scientific documents referred to in the reference list or in the scientific literature. For example, one may obtain VEGF or FGF-2 and FGF-5 segments by using molecular biological techniques, such as polymerase chain reaction (PCR), or by screening a cDNA or genomic library, using primers or probes with sequences based on the above nucleotide sequence. The practice of such a technique is a routine matter for those skilled in the art, as taught in various scientific articles, such as Sambrook et al., incorporated herein by reference. The angiogenetic genes and DNA segments that are particularly preferred for use in the present compositions and methods are VEGF, FGF-2 and FGF-5. It is also contemplated that one may clone further genes or cDNA that encode an angiogenic protein or polypeptide. The techniques for cloning DNA, i.e. obtaining a specific coding sequence from a DNA library that is distinct from other portions of DNA, are well known in the art. This can be achieved by, for example, screening an appropriate DNA library. The screening procedure may be based on the hybridisation of oligonucleotide probes, designed from a consideration of portions of the amino acid sequence of known DNA sequences encoding related angiogenic proteins. The operation of such screening protocols are well known to those skilled in the art and are described in detail in the scientific literature, for example Sambrook et al. (Sambrook et al., Molecular Cloning: a Laboratory Manual, 1989, Cold Spring Lab Press; Inniste et al., PCR strategies, 1995, Academic Press, New York).

Angiogenic genes, with sequences that vary from those described in the literature, are also encompassed by the invention, as long as the altered or modified gene still encodes a protein that functions to stimulate surrounding tissues of cardiovascular or tissue implants, in any direct or indirect manner. These sequences include those caused by point mutations, those due to the degeneracies of the genetic code or naturally occurring allelic variants, and further modifications that have been introduced by genetic engineering such as a hybrid gene, i.e. by the hand of man.

Techniques for introducing changes in nucleotide sequences that are designed to alter the functional properties of the encoded proteins or polypeptides are well known in the art.

Such modifications include the deletion, insertion or substitution of bases, and thus, changes in the amino acid sequence. Changes may be made to increase the angiogenic activity of a protein, to increase its biological stability or half-life, to decrease its degradation, increase its secretion, change its glycosylation pattern, and the like. All such modifications of the nucleotide sequences are encompassed by this invention.

It will also be understood that one, or more than one, angiogenic gene may be used in the methods and compositions of the invention. The nucleic acid delivery may thus entail the administration of one, two, three, or more angiogenic genes. The maximum number of genes that may be applied is limited only by practical considerations, such as the effort involved in simultaneously preparing a large number of gene constructs or even the possibility of eliciting an adverse cytotoxic effect. The particular combination of genes may be two or more angiogenic genes, or it may be such that a growth factor gene is combined with a hormone gene. A hormone or growth factor gene may even be combined with a gene encoding a cell surface receptor capable of interacting with a polypeptide product of the first gene. Also, an angiogenic gene can be combined with genes encoding antisense products. In using multiple genes, the genes may be combined on a single genetic construct under control of one or more promoters, or they may be prepared as separate constructs of the same or different types. Thus, an almost endless combination of different genes and genetic constructs may be employed. Certain gene combinations may be designed to, or their use may otherwise result in, achieving synergistic effects on angiogenesis and endothelialisation. Any of all those combinations are intended to fall within the scope of the present invention. Indeed, many synergistic effects have been described in the scientific literature, whereby a person skilled in the art readily would be able to identify likely synergistic gene combinations or even gene protein combinations. Also, another gene with qualities reducing thrombogenicity, fibrosis, or neointimal growth may be chosen. Another gene may encode a protein that inhibits the growth of neointimal cells, for example inducible nitric oxide synthase (iNOS) or endothelial cell nitric oxide synthase (ecNOS). Proteins or products of enzyme proteins that inhibit thrombosis, e.g. prostacyclin, tissue plasminogen activator (tPA), urokinase, and streptokinase, are also of interest for co-transfection. Also angiogenic genes may be combined with other genes which later inhibit the overexpression of angiogenic factors at any level such as transcription or translation. Administration may occur before, simultaneously or after administration of the angiogenic nucleic acid.

It will also be understood that the nucleic acid or gene could, if desired, be administered in combination with further agents, such as, e.g. proteins, polypeptides, aptamer oligonucleotides, transcription factor decoy oligonucleotides or various pharmacologically active agents, growth factors stimulating angiogenesis, adhesion molecules like fibronectin, substances such as heparin to promote endothelialisation etc. Also immunosuppressants and anti-inflammatory and anti-restenosis substances may be used. As long as genetic material forms part of the composition, there is virtually no limit for including other components, given that the additional agent does not cause a significant adverse effect upon contact with the target cells or tissues. The nucleic acids may thus be delivered along with various other agents. Also, nucleic acid may be delivered along with an implant giving radiation to the surrounding tissue to excert a specific effect along with angiogenesis.

It will also be understood that the nucleic acid or gene can be administered in combination with a simultaneous cell seeding or sodding procedure, and it can also be combined with simultaneous seeding or sodding with genetically modified cells.

### Gene constructs and nucleic acids:

As used herein, the terms gene and nucleic acid are both used to refer to a DNA molecule that has been isolated, and are free of total genomic DNA of a particular species. Therefore, a gene or a DNA encoding an angiogenic gene refers to a DNA that contains sequences encoding an angiogenic protein, but it is isolated from, or purified free from, total genomic DNA of the species from which the DNA is obtained. Included within the term DNA are DNA segments and smaller fragments of such segments, and also recombinant vectors, including for example plasmids, cosmids, artificial chromosomes, phages, lentivirus, retroviruses, adenoviruses, and the like.

The term gene is used for simplicity to refer to a functional protein- or peptide-encoding unit. As will be understood by those skilled in the art, this functional term includes both genomic sequences and cDNA sequences. Of course, this refers to the DNA segment as originally isolated, and does not exclude genes or coding regions, such as sequences encoding leader peptides or targeting sequences, later added to the segment by man.

This invention provides novel ways to utilise various known angiogenic DNA segments and recombinant vectors. Many such vectors are readily available. However, there is no requirement for a highly purified vector to be used, as long as the coding segment employed encodes an angiogenic protein, and does not include any coding or regulatory sequences that would have an adverse effect on the tissue surrounding the cardiovascular or tissue implant. Therefore, it will also be understood that useful nucleic acid sequences may include additional residues, such as additional non-coding sequences flanking either of the 5' or 3' portions of the coding region or may include various internal sequences, i.e. introns, which are known to occur within genes.

After the identification of an appropriate angiogenic gene or DNA molecule, it may be inserted into any one of the many vectors currently known in the art. In that way it will direct the expression and production of the angiogenic protein when incorporated into a tissue surrounding the implant. In a recombinant expression vector, the coding portion of the DNA segment is positioned under the control of a promoter. The promoter may be in a form that is naturally associated with an angiogenic gene. Coding DNA segments can also be positioned under the control of a recombinant, or heterologous, promoter. As used herein, a recombinant or heterologous promoter is intended to refer to a promoter that is not normally associated with an angiogenic gene in its natural environment. Such promoters may include those normally associated with other angiogenic genes, and/or promoters isolated from any other bacterial, viral, eukaryotic, or mammalian cell. Naturally, it will be important to employ a promoter that effectively directs the expression of the DNA segment in tissues surrounding the vascular prosthesis. The use of recombinant promoters to achieve protein expression is generally known to those skilled in the art of molecular biology (Sambrook et al.). The promoters used may be constitutive, or inducible, and can be used under the appropriate conditions to direct high level or regulated expression of the introduced DNA segment. The currently preferred constitutive promoters are for example CMV, RSV LTR, immunoglobulin promoter, SV40 promoter alone, and the SV40 promoter in combination with the SV40 enhancer, and regulatable promoters such as the tetra-cyclin-regulated promoter system, or the metalothionine promoter. The promoters may or may not be associated with enhancers, where the enhancers may be naturally associated with the particular promoter or associated with a different promoter. A termination region is provided 3' to the growth factor coding region, where the termination region may be naturally associated with the cytoplasmic domain or may be derived from a different source. A wide variety of termination regions may be employed without adversely affecting expression. After various manipulations, the resulting construct may be cloned, the vector isolated, and the gene screened or sequenced to ensure the correctness of the construct. Screening can be done with restriction analysis, sequencing or alike.

Angiogenic genes and DNA segments may also be in the form of a DNA insert, which is located within the genome of a recombinant virus, such as, for example, recombinant adenovirus, adenoassociated virus (AAV) or retrovirus. To place the gene in contact with a tissue surrounding an implant, one would, in such embodiments, prepare the recombinant viral particles, the genome that includes the angiogenic gene insert, and simply contact the tissues surrounding the implant with the virus, whereby the virus infects the cells and transfers the genetic material. In some embodiments of the invention, one would attach virus in a composition to an implant, such as a vascular prosthesis, cardiovascular patch, stent graft or graft connector, and then contact the tissue surrounding the implant with the implant in site. The virus is released from the composition, whereby cells grow into the implant, thereby contacting the virus and allowing viral infection, which results in that the cells take up the desired gene or cDNA and express the encoded protein, which in turn results in angiogenesis and endothelialisation of the implant.

In a preferred embodiment, the methods of the invention involve to prepare a composition in which the angiogenic gene, genes, or DNA segments are attached to or are impregnated on a vascular prosthesis, a cardiovascular patch, a stent graft, a heart valve, a graft connector, or a tissue implant to form a vascular prosthesis-, a cardiovascular patch-, an endovascular graft-, a graft connector-, a heart valve- or a tissue implant-gene composition. Then the vascular prosthesis-, cardiovascular patch-, stent graft-, graft connector-,heart valve-, tissue implant-gene composition may be placed in contact with tissue surrounding the said cardiovascular or tissue implant. Vascular prosthesis-, cardiovascular patch-, stent graft-, heart valve-, graft connector-, tissue implant-gene compositions are all those in which a gene is adsorbed, absorbed, or otherwise maintained in contact with the said implant.

### 2. Nucleic acid transfer into cells of tissue surrounding an implanted device

Once a suitable vascular implant-gene composition has been prepared or obtained, all that is required for delivering the angiogenic gene to the surrounding tissue, is to place the cardiovascular implant-gene or tissue implant-gene composition surgically, or with the help of a catheter, in contact with the wished site in the body, with or without first wrapping it with the surrounding tissue. The methods are well known to a person skilled in the art. The angiogenic gene can also be administered to the tissue before, during or after implanting the cardiovascular or tissue implant to the site. This could be an arteriovenous fistula, arterial bypass graft or interposition graft, a venous graft, cardiovascular patch, artificial heart, stent graft, stent, heart valve, cardiac asssist device, anastomotic device, annuloplasty, vascular catheter, pacemaker wire, tracheal cannula, biomedical sensor, chamber for living cells, artificial organ, organ implant, orthopedic implant, suture material, surgical patch, clip or pledget, or any medical device, all of which comprise at least one synthetic surface.

In the present invention, one or more vectors are transferred to any surrounding tissue, which preferably is a mammalian tissue. Several publications have postulated the use of gene transfer for the treatment or prevention of diseases (Levine and Friedman, Curr Opin in Biotech 1991; 2: 840-44, Mulligan, Science 1993; 260: 926-32, Crystal, Science 1995; 270:404-410, Rowland, Ann Thorac Surgery 1995;60:721-728; Nabel et al, Science 1990; 249: 1285-88). The eukaryotic host cell is optimally present *in vivo*. According to the present invention, the contacting of cells with the vectors of the present invention can be by any means by which the vectors will be introduced into the cell. Such introduction can be by any suitable method. Preferably, the vectors will be introduced by means of transfection, i.e. using the natural capability of the naked DNA to enter cells (e.g., the capability of the vector to undergo receptor-mediated endocytosis). However, the vectors can also be introduced by any other suitable means, e.g. by transduction, calcium phosphate-mediated transformation, microinjection, electroporation, osmotic shock, and the like.

The method can be employed with respect to various cells, differing both in number of vector receptors as well as in the affinity of the cell surface receptors for the vector. According to the invention, the types of cells to which gene delivery is contemplated *in vivo* include all mammalian cells, more preferably human cells. The vectors can be made into the compositions appropriate for contacting cells with appropriate (e.g. pharmaceutically acceptable) excipients, such as carriers, adjuvants, vehicles, or diluents. The means of making such a composition, and means of administration, have been described in the art. Where appropriate, the vectors can be formulated into preparations in solid, semisolid, liquid, or aerosol forms, such as aerosol, spray, paste, ointment, gel, glue, powders, granules, solutions, injections, creme and drops, in the usual ways for their respective route of administration without excluding any other method. A pharmaceutically acceptable form, that does not ineffectuate the compositions of the present invention should be employed. In pharmaceutical dosage forms, the compositions can be used alone or in an appropriate association, as well as in combination with other pharmaceutically active compounds. For example, nucleic acids encoding for VEGF can be administered together with nucleic acids encoding for inhibiting platelet deposition or smooth muscle cell proliferation. Accordingly, the pharmaceutical composition of the present invention can be delivered via various ways and to various sites in a mammalian to achieve a particular effect. A person skilled in the art will recognise that although more than one way can be used for administration, a particular way can provide a more immediate and more effective reaction than the other way. Local delivery can be accomplished by administration comprising topical application or instillation of the formulation on the implant, or administration of the formulation directly, to the tissues surrounding the implant *in vivo*, or any other topical application method. Administration of the drug this way, enables the drug to be site-specific, in a way that release of high concentrations and/or highly potent drugs may be limited to direct application to the targeted tissue. Preferred methods is to deliver nucleic acids in an aqueous solution incorporated in fibrin, hydrogel, glycosaminoglycans, glycopolysaccharides, or any other biocompatible polymeric carrier matrix, such as alginate, collagen, hyaluronic acid, polyurethane, cellulose, polylactic acid which covers at least a portion of the implant (5,833,651). Nucleic acids can be added to the polymer-coated implant, either at the time of implant manufacture or by the physician prior to, during or after implantation. Fibrin has a number of features that make it particularly suited for sustained gene delivery. Fibrin has holes, gaps and spaces that support and provide room for the nucleic acid. After implantation, the nucleic acid moves from the fibrin mesh to the tissues surrounding the implant. Fibrin is capable of dehydration and rehydration, which makes a fibrin covered implant suitable for loading nucleic acid in a liquid suspension. Fibrin is also biodegradable and fibrin biodegradation on a fibrin/nucleic acid implant further facilitates nucleic acid contact with the surrounding tissue. The polymer composition comprising fibrin and vector provide stabilising composition for gene delivery. The polymer may also be either a biostable or a bioabsorbable polymer, depending on the desired rate of release or the desired degree of polymer stability. It may be naturally occuring or synthetic compound, also derivatives and salts of the compounds are included. A bioabsorbable polymer is more desirable, as it causes no chronic local response. Bioabsorbable polymers that may be used include, but are not limited to, poly(L-lactic acid), polycaprolactone, poly(lactide-coglycolide), poly(hydroxybutyrate), poly(hydroxybuturate-co-valerate), polydioxanone, polyorthoester, polyanhydride, poly(glycolic acid), poly(D,L-lactic acid), polylactic-polyglycolic acid, polyglactin, polydioxone, polygluconate, poly(glycolic acid-cotrimethylene carbonate), polyphosphoester, polyphosphoester urethane, poly(amino acids), cyanoacrylates, poly(trimethylene carbonate), poly(iminocarbonate), eopoly(ether-esters)(e.g. PEO/PLA), polyalkylene oxalates, polyphosphazenes, and biomolecules, such as fibrin, fibrinogen, cellulose, starch, collagen, mucin, fibronectin, and hyaluronic acid. Also, biostable polymers with a relatively low chronic tissue response, such as polyurethanes, silicones, and polyesters could be used if they can be dissolved and cured or polymerised on the implant, such as polyelolefins, polyisobutylene and ethylene-alphaolefin copolymers; acrylic polymers and copolymers, vinyl halide polymers and copolymers, such as polyvinyl chloride; polyvinyl ethers, such as polyvinyl methyl ether; polyvinylidine halides, such as polyvinylidene fluoride and polyvinylidene chloride; polyacrylonitrile, polyvinyl ketones; polyvinyl aromatics, such as polystyrene, polyvinyl esters, such as polyvinyl acetate; copolymers of vinyl monomers with each other and olefins, such as ethylene-methyl methacrylate copolymers, acrylonitrile-styrene copolymers, ABS resins, ethylene-vinyl acetate copolymers: polyamides, such as Nylon 66 and polycaprolactam;alkyd resins; polycarbonates; polyoxymethylenes; polyimides; polyethers; epoxy resins; polyurethanes; rayon; rayon-triacetate; cellulose, cellulose acetate, cellulose butyrate; cellulose acetate butyrate; cellophane; cellulose nitrate; cellulose propionate; cellulose ethers; and carboxymethylcellulose (5,776,184). Also fibrin together with other biocompatible polymers, either natural or synthetic and their derivatives and salts, may be used. Of the polymers glycopolysaccharides-may advantageous. In one aspect there is a solid/solid solution of polymer and drug. This means that the drug and the polymer both are soluble in the same solvent and have been intimately admixed in the presence of that solvent. The drug and polymer can be applied in various ways, such as by simply immersing the implant into the solution or by spraying the solution onto the implant (5,776,184). Various hydrogel polymers can be used, such as those selected from the group consisting of polycarboxylic acids, cellulosic polymers, gelatin, alginate, poly 2-hydroxyethylmethylacrylate (HEMA) polyvinylpyrrolidine, maleic anhydride polymers, polyamids, polyvinyl alcohols, polyethylene oxides, polyethylene glycol, polyacrylamide, polyacids, e.g. polyacrylic acids, polysaccharide, e.g. a mucopolysaccharide such as hyaluronic acid (5,674,192)(5,843,089). The polymer can be porous or nonporous on the implant. Several layers of polymers can be utilised and several different polymers can be combined on the same implant. Different layers and different polymers can carry different pharmacological substances (5,833,651). Also, one or more surfaces of the implant can be coated with one or more additional coats of polymer that is the same or different from the second polymer. The adhesion of the coating and the rate at which the drug is delivered can be controlled by selection of an appropriate bioabsorbable or biostable polymer, and by the ratio of drug to polymer in the solution (5,776,184). The dosage applied to the tissue may also be controlled by regulating the time of presoaking drug into the hydrogel coating to determine the amount of absorption of the drug solution by the hydrogel coating. Other factors affecting the dosage are the concentration of the drug in the solution applied to the coating, and the drugreleasability of the hydrogel coating, determined by, for example, the thickness of the hydrogel coating, its resiliency, porosity and the ability of the hydrogel coating to retain the drug, e.g. electrostatic binding or pore size, or the ionic strength of the coating, e.g. changed by changing the pH. It may be advantageous to select a hydrogel coating for a particular drug, such that the drug is not substantially released into body fluids prior to application to the site. The release of the solid/solid solution of polymer and drug can further be controlled by varying the ratio of drug to polymer in the multiple layers. Coating need not be solid/solid solution of polymeric and drug, but may instead be provided from any combination of drug and polymer applied to implant. The ratio of therapeutic substance to polymer in the solution will depend on the efficacy of the polymer in securing the therapeutic substance onto the implant and the rate at which the coating is to release the therapeutic substance to the tissues. More polymer may be needed if it has a relatively poor efficacy in retaining the therapeutic substance on the implant, and more polymer may be needed in order to provide an elution matrix that limits the elution of a very soluble therapeutic substance. Therefore, a wide therapeutic substance-to-polymer rate could be appropriate, and it could range from about 10:1 to 1:100 (5,776,184). Binding of the drug may also be accomplished by electrostatic attraction of the drug to the coating or to a coating additive or a mechanical binding, for example by employing a coating having a pore size that inhibits inward flow of body fluids or outward flow of the drug itself, which might tend to release the drug.

Hydrogels are particularly advantageous in that the drug is held within the hydrogen-bond matrix formed by the gel (5,674,192). Examples of hydrogels are for example HYDRO-PLUS.RTM (5,674,192), CARBOPOL.RTM (5,843,089), AQUAVENE.RTM (4,883,699), HYPAN.RTM (4,480,642). In some cases, the hydrogel may be crosslinked prior to lining the implant, for example the hydrogel coating on a vascular or endovascular graft may be contacted with a primer,dip before the hydrogel is deposited on the implant. If crosslinked it forms a relatively permanent lining on the implant surface, and if left uncrosslinked it forms a relatively degradable lining on the implant surface. For example, the longevity of a crosslinked form of a given hydrogel in the stent lining, has been at least twice to that of its uncrosslinked form (5,843,089). Alternatively, the hydrogel lining may be contacted with a crosslinking agent *in situ* (5,843,089). In general, when dry, the hydrogel coating is preferably on the order of about 1 to 10 microns thick, and typically of 2 to 5 microns. Very thin hydrogel coatings, e.g., of about 0,2-0,3 microns (dry) and much thicker hydrogel coatings, e.g., more than 10 microns (dry) are also possible. Typically, the hydrogel coating thickness may swell with a factor of about 6 to 10 or more, when hydrogel is hydrated (5,674,192). Usually, the polymeric carrier will be biodegradable or bioeluting (taught for example by 5,954,706, 5,914,182, 5,916,585, 5,928,916). The carrier can also be constructed to be a biodegradable substance filling the pores, and release one or more substances into the surrounding tissue by progressive dissolution of the matrix. Subsequently the pores will open. The delivered vectors may be nucleic acids encoding for therapeutic protein, e.g. a naked nucleic acid or a nucleic acid incorporated into a viral vector or liposome: By a naked nucleic acid is meant an single or double stranded DNA or RNA molecule not incorporated into a virus or liposome. Antisense oligonucleotides which specifically bind to complementary mRNA molecules, and thereby reduce or inhibit protein expression, can also be delivered to the implant site via the hydrogel coating (5,843,089). Generally, attachment of the nucleic acid to the implant can also be done in several other ways, such as by using covalent or ionic attachment techniques. Typically, covalent attachment techniques require the use of coupling agents, such as glutaraldehyde, cyanogen bromide, p-benzoquinone, succinic anhydrades, carbodiimides, diisocyanates, ethyl chloroformate, dipyridyl disulphide, epichlorohydrin, azides, among others, without excluding any other agent, but any method that uses the described methods of this invention can be used and will be recognised by a person skilled in the art. Covalent coupling of a biomolecule to a surface may create undesirable crosslinks between biomolecules, and thereby destroying the biological properties of the biomolecule. Also, they may create bonds amongst surface functional sites and thereby inhibit attachment. Covalent coupling of a biomolecule to a surface may also destroy the biomolecules three-dimensional structure, and thereby reducing or destroying the biological properties (5,928,916). Ionic coupling techniques have the advantage of not altering the chemical composition of the attached biomolecule, and ionic coupling of biomolecules also has an advantage of releasing the biomolecule under appropriate conditions. One example is (4,442,133). The current techniques for immobilisation of biomolecules by an ionic bond have been achieved by introducing positive charges on the biomaterial surface utilising quaternary ammonium salts, polymers containing tertiary and quaternary amine groups, such as TDMAC, benzalconium chloride, cetylpyrridinium chloride, benzyldimethylstearyammonium chloride, benzylcetyidimethylammonium chloride, guanidine or biguanide moiety (5,928,916). When delivering the vascular implant percutaneously, a sheath member may be included to inhibit release of the drug into body fluids during placement of the catheter. For example, it can be carbowax, gelatin, polyvinyl alcohol, polyethylene oxide, polyethylene glycol, or a biodegradable or thermally degradable polymer, e.g. albumin or pluronic gel F-127 (5,674,192). The particular type of attachment method when practising the methods and compositions of the invention is not important, as long as the nucleic acids released from the implant stimulates the surrounding tissue in such a way that they are activated and, in the context of *in vivo* embodiments, ultimately give rise to endothelialisation of the cardiovascular or tissue implant without causing adverse reactions. The methods described herein are by no means all inclusive, and further methods to suit the specific application will be apparent to the skilled person of the art.

The composition useful in the present invention can be provided in unit dosage form, wherein each dosage unit, e.g. solution, gel, glue, drops and aerosol, contains a predetermined amount of the composition, alone or in appropriate combination with other active agents. The term unit dosage form, as used herein, refers to physically discrete units suitable as unitary dosages for human and animal subjects, whereby each unit contains a predetermined quantity of the compositions of the present invention, alone or in combination with other active agents, calculated in an amount sufficient to produce the desired effect, in association with a pharmaceutically acceptable diluent, carrier, or vehicle, where appropriate. The specifications for the unit dosage forms of the present invention depend on the particular effect to be achieved, and the particular pharmacodynamics associated with the pharmaceutical composition in the particular host.

Accordingly, it is disclosed a method of transferring a therapeutic gene to a host, which comprises administering the vector described in the present invention, preferably as a part of composition with the implant, using the aforementioned ways of administration or alternative ways known to those skilled in the art. The effective amount of the composition is such as to produce the desired effect in a host, which can be monitored using several end-points known to those skilled in the art. Effective gene transfer of a vector to a host cell, can be monitored in terms of a therapeutic effect (e.g. formation of capillaries and endothelialisation of surfaces), or further by evidence of the transferred gene or expression of the gene within the host (e.g. using the polymerase chain reaction in conjunction with sequencing, Northern or Southern hybridisations, or transscription assays to detect the nucleic acid in host cells, or using immunoblot analysis, antibody-mediated detection, mRNA or protein half-life studies, or particularised assays to detect protein or polypeptide encoded by the transferred nucleic acid, or impacted in level or function due to such transfer). One such particularised assay described in the examples includes Western immunoassay for detection of proteins encoded by the VEGF-gene. These methods are by no means all-inclusive, and further methods to suit the specific application will be apparent to a person skilled in the art. Moreover, the effective amount of the compositions can be further approximated through analogy to compounds known to exert the desired effect (e.g., compounds traditionally employed to stimulate angiogenesis can provide guidance in terms of the amount of a VEGF and FGF-5 nucleic acid to be administered to a host).

Furthermore, the preferred amounts of each active agent included in the compositions according to the invention, VEGF is preferably included from about 0.1 micrograms to 10000 micrograms (although any suitable amount can be utilised either above, i.e. greater than about 10000 micrograms, or below, i.e. less than about 0.1 micrograms), provide general guidance of the range of each component to be utilised by the practitioner upon optimising the methods of the present invention for practice *in vivo*. Similarly, FGF-5 and FGF-2 plasmids are included from 0.1 to 10000 micrograms (although any suitable amount can be utilised either above, i.e. greater than about 10000 micrograms, or below, i.e. less than about 0.1 micrograms). The FGF-5 vector preferably has between 10⁷ and 10¹³ viral particles, although any suitable amount can be utilised, either more than 10¹³ or less than 10⁷. Moreover, such ranges by no means preclude use of a higher or lower amount of a component, as might be warranted in a particular application. For instance, actual dose and schedule can vary depending on whether the compositions are administered in combination with other pharmaceutical compositions, or depending on interindividual differences in pharmacokinetics, drug disposition, and metabolism. Furthermore, the amount of vector to be added per cell will likely vary with the length and stability of the gene inserted in the vector, as well as also the nature of the sequence, and is particularly a parameter which needs to be determined empirically, and it can be altered due to factors not inherent to the methods of the present invention (for instance, the cost associated with synthesis). A person skilled in the art can easily make any necessary adjustments in accordance with the exigencies of the particular situation. The amount of gene construct that is applied to the surrounding tissue or the amount of gene composition that is applied on the implant or in the tissue, will be finally determined by the attending physician or veterinarian considering various biological and medical factors. For example, one would wish to consider the particular angiogenic gene and vascular implant material, patient or animal size, age, sex, diet, time of administration, as well as any further clinical factors that may affect endothelialisation, such as serum levels of different factors and hormones. The suitable dosage regimen will therefore be readily determinable by a person skilled in the art in light of the coming disclosure, bearing the individual circumstances in mind.

Also, for these embodiments, when one or more different vectors (i.e. each encoding one or more different therapeutic genes) are employed in the methods described herein, the contacting of cells with various components of the present invention can occur in any order or can occur simultaneously. Preferably it occurs simultaneously.

### 3. Endothelialisation promoting tissue

This invention provides advantageous methods for using genes to stimulate porous medical implant endothelialisation from surrounding tissue. As used here surrounding tissue refers to any or all of those cells that have the capacity to ultimately form, or contribute to the formation of, new endothelium into the implant surface. This includes various tissues in various forms, such as for example pleura, pericardium, peritoneum, omentum, fat and muscle.

The particular type or types of surrounding tissue, which are stimulated with the methods and compositions of the invention, are not important, as long as the cells are stimulated in such a way that they are activated, and, in the context of *in vivo* embodiments, ultimately give rise to endothelialisation or capillarisation of the implant.

The surrounding tissue is also used to particularly refer to those cells that are located within, are in contact with, or migrate towards the implant, and which cells directly or indirectly stimulate the formation of endothelium and/or capillaries. As such, microvascular endothelial cells may be cells that form endothelium. Cells, that upon stimulation further attract endothelial cells, are also considered to be surrounding tissue in the context of this disclosure, as their stimulation indirectly leads to endothelialisation. Cells affecting endothelialisation indirectly may do so by the elaboration of various growth factors and cytokines, or by their physical interaction with other cell types. Also, cells or tissues that in their natural environment arrive at an area of active implant endothelialisation or vascularisation may be surrounding tissue. Surrounding tissue cells may also be cells that are attracted or recruited to such an area. Although of scientific interest, the direct or indirect mechanisms by which surrounding tissue cells stimulate endothelialisation is not a consideration in the practising of this invention.

Surrounding tissue cells may be cells or tissues that in their natural environment arrive at an area of active vascular prosthesis, endovascular prosthesis endothelialisation, or tissue implant vascularisation. In terms of surrounding tissue, these cells may also be cells that are attracted or recruited to such an area.

According to the invention, the surrounding cells and tissues will be those cells and tissues that arrive to the surface of cardiovascular implants that one wishes to endothelialise, or cells or tissues that arrive to the surface of tissue implants that one wants to vascularise.

Accordingly, in treatment embodiments there is no difficulty associated with the identification of suitable surrounding tissues to which the present therapeutic compositions, and cardiovascular and tissue implants should be applied. All that is required in such cases is to obtain an appropriate stimulatory composition, as disclosed herein, and to contact the cardiovascular or tissue implant with the stimulatory composition and the surrounding tissue. The nature of this biological environment is such that the appropriate cells will become activated in the absence of any further targeting or cellular identification by the practitioner.

One aspect of the invention involves to generally contact surrounding tissues with a composition comprising one or two genes (with or without additional genes, proteins, growth factors, drugs or other biomolecules), and a cardiovascular or tissue implant to promote expression of said gene in said cells. As outlined, cells may be contacted *in vivo.* This is achieved, in the most direct manner, by simply obtaining a functional endothelialisation promoting gene construct, and applying the construct to the cells. Contacting the cells with DNA, e.g. a linear DNA molecule, or DNA in the form of a plasmid, or some other recombinant vector that contains the gene of interest under the control of a promoter, along with the appropriate termination signals, is sufficient to achieve an uptake and an expression of DNA, with no further steps necessary.

Preferably the process of contacting the surrounding tissue with the endothelialisation promoting composition is conducted *in vivo*. Again, a direct consequence of this process is that the cells take up and express the gene, and the translational or the transcrigtional product stimulates the process of angiogenesis resulting in endothelialisation and/or capillarisation of the implant without additional steps required by the practitioner.

### 4. Materials used in the devices according to the invention

As used herein, the following terms and words shall have the following ascribed meanings. Implantable medical device, which for brevity will be referred to as implant, device or prosthesis will refer to an object that is fabricated, at least in part, from a biomaterial, and is intended for use in contact with bodily tissues, including bodily fluids. Biomaterial shall refer to the composition of the material used to prepare a device, which provides one or more of its tissue contacting surfaces. Porosity and inflections thereof (such as pores and porous), if not specified otherwise, shall refer to a biomaterial having small channels or passages which start at an external (e.g. first major) surface of the biomaterial and extend substantially through the biomaterial to an internal (e.g., second) surface. Rigid and inflections thereof, will, in case of a nonabsorbable biomaterial, when fabricated in the form of an implantable medical device, refer to the ability to withstand the pressures encountered in the course of its use, e.g. to retain patency and pore structure *in vivo*. The surface shall refer to the interface between the biomaterial and its environment. The term is intended to include the use of the word in both its macroscopic sense (e.g. the two major faces of a sheet of biomaterial), as well as in its microscopic sense (e.g. the lining of pores traversing the material). The term "attach" and its derivatives refer to adsorption, such as physisorption, or chemisorption, ligand/receptor interaction, covalent bonding, hydrogen bonding, or ionic bonding of a polymeric substance or nucleic acids to the implant. Endothelialisation will, unless otherwise specified, be used interchangeably with the phrase capillary endothelialisation to refer to the growth of endothelial cells on substantially all tissue contacting surfaces of a biomaterial used to form a porous rigid or nonporous rigid implant.

The type of cardiovascular and tissue implants that may be used in the compositions, devices and methods of the invention is virtually limitless, as long as they are tissue compatible. Thus, devices of the present invention include medical devices intended for prolonged contact with blood, bodily fluids or tissues, and in particular, those that can benefit from the capillary endothelialisation when used for *in vivo* applications. Preferred devices are implantable in the body, and include cardiovascular implants, tissue implants, artificial organs, such as the pancreas, liver, and kidney, and organ implants, such as breast, penis, skin, nose, ear and orthopedic implants. The significance of capillary endothelialisation will vary with each particular device, depending on the type and purpose of the device. Ingrown capillaries can provide endothelial cells to line surfaces of vascular implants, protect tissue implants from infection, carry nutrients to the cells in the device and make it possible for sensors to sense substance levels in circulation. This means that the implant has all the features commonly associated with biocompatibility, in that they are in a form that does not produce an adverse, an allergic, or any other untoward reaction when administered to a mammal. They are also suitable for being placed in contact with the tissue surrounding the implant. The latter requirement takes factors, such as the capacity of the said implants to provide a structure for the developing vascular endothelium, into consideration.

Preferred biomaterials are those that provide sufficient rigidity for their intended purposes *in vivo.* For use in forming a vascular graft and cardiovascular patch, for instance, the biomaterial will be of sufficient rigidity to allow the graft to retain graft patency in the course of its intended use. The choice of implant material will differ according to the particular circumstances and the site where the vascular or tissue implant'is implanted. Vascular prosthesises are made of biomaterials, selected from the group consisting of e.g. tetrafluoroethylene polymers, aromatic/aliphatic polyester resins, polyurethans, and silicone rubbers. However, any type of biocompatible microporous mesh may be used. The said biomaterials can be combined with each other or other substances, such as polyglycolic acid, polylactic acid, polydioxone and polyglyconate. Preferred are expanded polytetrafluorethylene and Dacron. Dacron may be with or without velour, or modified in some other way. Dacron is usually woven, braided or knitten and suitable yarns are between 10 and 400 deniers. The nodal regions of ePTFE are composed of nonporous PTFE that serves to provide tear resistance (e.g. for sutures and resistance to aneurysmal dilatation). The internodal regions are composed of fibers of PTFE, which serve to connect the nodes with the spaces between the fibers providing the porosity referred to herein. The nodal size can be expressed as the percentage of the tissue-contacting surface that is composed of nodal PTFE. The distance between nodes can be expressed as the average fibril length. In turn, the porosity is commonly expressed as the internodal distance (i.e. the average distance from the middle of one node to the middle of the adjacent node). Preferred ePTFE materials have nodes of sufficient size and frequency to provide adequate strength (e.g., with respect to aneurysmal dilatation) and internodal regions of sufficient frequency and fiber length to provide adequate porosity (to allow for capillary endothelialisation). Given the present specification, those skilled in the art will be able to identify and fabricate devices using biomaterials having a suitable combination of porosity and rigidity. Biomaterials are preferably porous to allow the attachment and migration of cells, which may be followed by the formation and growth of capillaries into the surface. Suitable pores can exist in the form of small channels or passages, which start at an external surface and extend partially or completely through the biomaterial. In such cases, the cross sectional dimensions of the pore capillary diameter are greater than 5 microns and typically less than 1 mm. The upper pore size value is not critical as long as the biomaterial retains sufficient rigidity, however it is unlikely that useful devices would have a pore size greater than about 1mm. Such pore dimensions can be quantified in microscope. As will be understood by those skilled in the art, several modifications of the graft materials and surfaces can be made, such as precoating with, for example, proteins (see e.g. 5,037,377,4,319,363), non-heparinised whole blood and platelet rich plasma, glow-discharge modifications of surfaces, adding pluronic gel, fibrin glue, fibronectin, adhesion molecules, covalent bonding, influencing surface charges, with for example carbon (5,827,327, 4,164,045), and treating with a surfactant or cleaning agent, without excluding any other method. Moreover, the implant can be constructed as a hybrid of different internodal distances for the inner and outer surfaces, such as 60 microns as an outer value and 20 microns as an inner value, for the internodal distances (HYBRID PTFE). Also, more layers with different internodal distances may be used. They are all intended to fall within the scope of the present invention when not inhibiting endothelialisation. Potential biodegradable vascular implants may be used in connection with the compositions, devices and methods of this invention. For example, biodegradable and chemically defined polylactic acid, polyglycolic acid, matrices of purified proteins, semi-purified extracellular matrix compositions and also collagen can be employed. Also, naturally occuring autogenic, allogenic and xenogenic material, such as an umbilical vein, saphenous vein, native bovine artery or intestinal sub-mucosal tissue may be used as a vascular implant material. Examples of clinically used grafts are disclosed in 4,187,390, 5,474,824 and 5,827,327. Biodegradable or bioabsorbable materials, such as homopolymers e.g. poly-paradioxanone, polylysine or polyglycolic acid and copolymers; e.g., polylactic acid and polyglycolic acids or other bio materials, may be used either alone or in combination with other materials as the vascular graft material, as long as they provide the required rigidity. Also, other biological materials, such as intestinal submucosa, matrices of purified proteins and semi-purified extracellular matrix compositions may be used. Appropriate vascular grafts will both deliver the gene composition and also provide a surface for new endothelium growth, i.e., will act as an *in situ* scaffolding through which endothelial cells may migrate. It will be understood by a person skilled in the art that any material with biocompatibility, rigidity and porosity to allow transgraft growth will be acceptable.

Background for cardiovascular patches is well described in for example 5,104,400, 4,164,045,5,037,377. In the case of vascular patches, one side of the patch engages the blood while the other side engages other surrounding tissues to promote transgraft growt of the endothelial cells. In the case of intracardiac patches, blood engages both sides of the patch. Preferred biomaterials are those that provide sufficient rigidity *in vivo.* A vascular patch biomaterial will be of sufficient rigidity to allow the patch to retain its form and porestructure in the course of its intended use. The choice of patch material will differ according to the particular circumstances and site where the vascular patch is implanted. Vascular patch is made of synthetic biomaterial, such materials include, but are not limited to, tetrafluoroethylene polymers, aromatic/aliphatic polyester resins, polyurethans, and silicone rubbers, however any type of biocompatible microporous mesh may be used. The said biomaterials can be combined with each other or other substances such as polyglycolic acid. Preferred are expanded polytetrafluorethylene and Dacron. Dacron is usually woven, braided or knitted, and with or without velour, and suitable yarns are between 10 and 400 deniers. The nodal regions of ePTFE are composed of nonporous FIFE that serves to provide tear resistance (e.g. for sutures and resistance to aneurysmal dilatation). The internodal regions are composed of fibers of PTFE which serve to connect the nodes, with the spaces between the fibers providing the porosity referred to herein. The nodal size can be expressed as the percentage of the tissue-contacting surface that is composed of nodal PTFE. The distance between nodes can be expressed as the average fibril length. In turn the porosity is commonly expressed as the internodal distance (i.e. the avarage distance from the middle of one node to the middle of adjacent node). Preferred ePTFE materials have nodes of sufficient size and frequency to provide adequate strength (e.g., with respect to aneurysmal dilatation) and internodal regions of sufficient frequency and fiber length to provide adequate porosity (to allow for capillary endothelialisation). Such materials will provide fewer though thicker nodes, which will in turn confer significantly greater strength *in vivo*. Given the present specification, those skilled in the art will be able to identify and fabricate devices using biomaterials having a suitable combination of porosity and rigidity. Biomaterials are preferably porous to allow the attachment and migration of cells, which may be followed by the formation and growth of capillaries into the luminal surface. Suitable pores can exist in the form of small channels or passages, which start at an external surface and extend through the biomaterial. In such cases, the cross sectional dimensions of the pores are larger than the diameter of a capillary 5 microns and are typically less than 1 mm. Upper pore size value is not critical as long as the biomaterial retains sufficient rigidity. However, it is unlikely that useful devices would have pore size greater than about 1mm. Such pore dimensions can be quantified in microscope. As will be understood by a person skilled in the art, several modifications of graft materials and surfaces can be made, such as precoating with for example proteins (for example, 5,037,377,4,319,363), non-heparinised whole blood and platelet rich plasma, glow-discharge modifications of surfaces, adding pluronic gel, fibrin glue, adhesion molecules, covalent bonding, influencing surface charges with for example carbon (5,827,327,4,164,045), treating with a surfactant or cleaning agent, without excluding any other method. Also the implant can be constructed as a hybrid of different internodal distances in inner and outer surface, such as outer 60 microns and inner 20 microns in internodal distance (HYBRID PTFE). Even more layers with different internodal distances may be used. They all are intended to fall in the scope of present invention when not inhibiting endothelialisation. Potential biodegradable materials may be used in connection with the compositions, devices and methods of this invention, for example homopolymers e.g. poly-paradioxanone, polylysine or polyglycolic acid and copolymers e.g., polylactic acid and polyglycolic acids or other bio materials, such as matrices of purified proteins and semi-purified extracellular matrix compositions may be used either alone or in combination with other materials as cardiovascular patch material, as long as they provide the required rigidity. Naturally occuring autogenic, allogenic and xenogenic material such as an umbilical vein, saphenous vein, native bovine artery, pericardium or intestinal submucosal tissue may also be used as cardiovascular patch material. Examples of clinically used vascular patches are disclosed in 5,037,377, 5,456,711, 5,104,400, 4,164,045. Appropriate vascular patches will both deliver the gene composition and also provide a surface for new endothelium growth, i.e., will act as an *in situ* scaffolding on which and through which endothelial cells may migrate. Preferably, nucleic acids are attached to the side engaging the tissues surrounding the vessel. Appropriate intracardiac patches will both deliver the gene composition to the surrounding tissues and provide a surface for new endothelium growth, i.e., will act as an *in situ* scaffolding on which and through which endothelial cells may migrate. Preferably, nucleic acids are attached to both intracardiac patch surfaces. Alternatively, nucleic acids may be attached to one of the intracardiac patch surfaces. It will be understood by a person skilled in the art that any material with biocompatibility, rigidity and porosity to allow endothelialisation will be acceptable.

Stent herein means a medical implant in the form of a hollow cylinder, which will provide support for the body lumen when it is implanted in contact with a site in the wall of a lumen to be treated. They can be of several different designs such as tubular, conical or bifurcated. The configuration can be such as a coiled spring, braided filament, perforated tube, slit tube, and zig-zag, or any other variant. Preferably, it is adapted for use in blood vessels in a way that the stent has an outer, lumen-contacting surface, and an inner, blood-contacting surface. Many stents of the art are formed of individual member(s), such as wire, plastic, metal strips, or mesh, which are bent, woven, interlaced or otherwise fabricated into a generally cylindrical configuration. The stent can also have underlying polymeric or metallic structural elements, onto which elements, a film is applied (5,951.586). Stents have been classified into either self-expanding or pressure expandable. The terms expand, expanding, and expandable are used herein to refer to diametrically adjustable intraluminal stents. When the self expanding stents are positioned at the treatment site with a delivery catheter, they are supposed to radially expand to a larger diameter after being released from a constraining force, which force restricts them to a smaller diameter and conform a surface contact with a blood vessel wall or other tissue without exertion of outwardly directed radial force upon stent. Stents of this type include stents of braided or formed wire. The presssure-expandable stents are fabricated of malleable or plastically deformable material, typically formed of metal wire or metal strips. The collapsed stent is taken to the treatment site with a delivery catheter, and is then radially expanded with a balloon or other stent-expansion apparatus to its intended operative diameter. Thread elements or strands formed of metal are generally favored, for applications requiring flexibility and effective resistance to radial compression after implantation. The favorable combination of strength and flexibility is largely due to the properties of the strands after they have been age hardened, or otherwise thermally treated in the case of polymeric strands. The braiding angle of the helical strands and the axial spacing between adjacent strands also contribute to strength and flexibility.

Stent wires may be of metal, inorganic fibers or organic polymers. They should be elastic, strong, biocompatible, and fatigue and corrosion resistant. For example, core wires made of metals, such as stainless steel or gold or other relatively pliable non-toxic metals and alloys that do not degrade during the time of implantation or are not subject to severe degradation (corrosion) under the influence of an electric current, are usually chosen. Such metals include, but are not limited to, platinum, platinum-iridium alloys, copper alloys, with tin or titanium, nickel-chrome-cobalt alloys, cobalt based alloys, molybdenium alloys, nickeltitanium alloys. The strands need not be of metal and may for example be of a polymeric material such as PET, polypropylene, PEEK, HDPE, polysulfone, acetyl, PTFE, FEP, and polyurethane without excluding any other substance (other variants: polytetrafluorethylene, fluorinated ethylene propylene, polytetrafluorethylene-perfluoroalkyl vinyl ether copolymer, polyvinyl chlorid, polypropylene, polyethylene terephthalate, broad fluoride and other biocompatible plastics). Also, a biodegradable or bioabsorbable material, such as homopolymers e.g. poly-paradioxanone, polylysine or polyglycolic acid and copolymers, e.g. polylactic acid and polyglycolic acids, polyurethane, or other biomaterials, may be used either alone or in combination with other materials as the stent material. Such monofilament strands range from 0,002 to 0,015 inches in diameter but of course the diameter could vary depending on the lumen size and the degree of support needed. To stents may also antithrombotic, anti-platelet, vasodilators, antiproliferative, antimigratory, antifibrotic, anti-inflammatory agents and more specifically, heparin, hirudin, hirulog, etritinate, freskolin and the like, be attached. Examples of clinically used stents are disclosed in 4,733,665, 4,800,882 and 4,886,062.

Stent grafts, also called covered stents, for transluminal implantations include a resilient tubular interbraided latticework of metal or polymeric monofilaments, a tubular interbraided sleeve formed of a plurality of interwoven textile strands, and an attachment component that fixes the latticework and the sleeve together, in a selected axial alignment with one another, engaged with one another and with a selected one of the latticework and the sleeve surrounding the other, whereby the latticework structurally supports the sleeve. It is ensured that the latticework and the sleeve behave according to substantially the same relationship governing the amount of radial reduction that accompanies a given axial elongation. The sleeve may be exterior or interior to the latticework, or the latticework may be integrated in the sleeve, and it can be continuous or discontinuous. Several prosthesis constructions have been suggested for composite braided structures that combine different types of strands, e.g. multifilament yarns, monofilaments, fusible, materials and collagens. Examples are found in WO91/10766. Textile strands are preferably multifilament yarns, even though they can be monofilaments. In either case the textile strands are much finer than the structural strands, ranging from about 10 denier to 400 denier. Individual filaments of the multifilament yarns can range from about 0,25 to about 10 denier. Multifilament yarns can be composed of various materials, such as PET, polypropylen, polyethylen, polyurethane, HDPE, silicone, PTFE, polyolefins and ePTFE. By modifying the yarns it is possible to modify sleeve qualities, for example untwisted flat filaments provide thinner walls, smaller intersticies between yarns so achieving lower permeability, and higher yarn cross-section porosity for capillary transgraft growth. Porous expanded PTFE film has a microstructure of nodes interconnected by fibrils and may be made as taught by for example U.S. Pat. Nos. 3,953,566, 4,187,390 and 4,482,516. Suitable pores can exist in the form of small channels or passages starting at an external surface and extending through the biomaterial. In such cases the cross-sectional dimensions of the pores are larger than the diameter of a capillary 5 microns, and are typically less than 1 mm. Upper pore size value is not critical so long as the biomaterial retains sufficient rigidity, however it is unlikely that useful devices would have pore size greater than about 1mm. Such pore dimensions can be quantified in microscope. As will be understood by those in the art several modifications of stent graft materials and surfaces can be made such as precoating with proteins, non-heparinised whole blood and platelet rich plasma, glow-discharge modifications of surfaces, adding pluronic gel, fibronectin, fibrin glue, adhesion molecules, covalent bonding, influencing surface charges with for example carbon (5,827,327, 4,164,045), treating with a surfactant or cleaning agent, mechanically changing the characteristics, such as adding grooves and changing the end angles without excluding any other method. Also the implant can be constructed as a hybrid of different internodal distances in inner and outer surface such as outer 60 microns and inner 20 microns in internodal distance (HYBRID PTFE). Even more layers with different internodal distances can be used. They all are intended to fall in the scope of present invention when not inhibiting endothelialisation. The fibrils can be uni-axially oriented, that is oriented in primarily one direction, or multiaxially oriented, that is oriented in more than one direction. The term expanded is used herein to refer to porous expanded PTFE. It will be understood by a person skilled in the art, that any material with biocompatibility and porosity to allow transgraft growth will be acceptable. Examples of clinically used stent grafts are disclosed in 5,957,974,5,928,279, 5,925,075, 5,916,264.

Also, naturally occuring autologous, allogenic or xenogenic materials, such as arteries, veins and intestinal submucosal can be used in stent grafts, such as an umbilical vein, saphenous vein, or native bovine artery. Potential biodegradable vascular implants may be used as stent grafts in connection with the compositions, devices and methods of this invention, for example biodegradable and chemically defined polylactic acid, polyglycolic acid, matrices of purified proteins, semi-purified extracellular matrix compositions. Appropriate vascular grafts and stent grafts will both deliver the gene composition and also provide a surface for new endothelium growth, i.e., will act as an *in situ* scaffolding through which endothelial cells may migrate. The particular design of the implants that are implanted using the methods and compositions of the invention are not important, as long as they act as scaffolds through which endothelium can migrate, in the context of *in vivo* embodiments, and ultimately give rise to endothelialisation of the implant.

A variety of catheter systems are useful for delivering the interventional stents and stent grafts into the desired site The chosen type is not important as long as the methods of present invention are used.

Heart valves are vell known in the art and operate hemodynamically as a result of the pumping action of the heart. Generally, there is an annular body having an interior surface, which defines a blood flow passageway, and which has one or multiple occluders supported thereon, for alternately blocking, and then allowing the blood flow in a predetermined direction. Heart valve prostheses are of various different designs, and of autologous, allogenic, xenogenic or synthetic material. The mechanical valve annular housing, also called annular body, and the valving members, can be made of any biocompatible and nonthrombogenic material, that also will take the wear they will be subjected to. There are various different designs, such as a circular valve housing and a valving member, such as a spherical member or ball, pivoting disc, poppet disc, and leaflet members, such as single or multiple leaflet constructs, for example two flat leaflets, leaflets with conical, semiconical and cylindrical surfaces. The orifice ring can be made of various materials, such as a pyrocarbon coated surface, a silver coated surface or from solid pyrolytic carbon (4443894), and leaflets may be made of one substrate, such as polycristalline graphite, plastic, metal or any other rigid material, and then coated with another, such as pyrolytic carbon (e.g. 3546711, 3579645). Circular valve housing can be porous, (here referred as having a porous surface and a network of interconnected interstitial pores below the surface in fluid flow communication with the pores, see 4,936,317), or nonporous, and suitable means, such as peripheral groove or a pair of flats can be provided for attaching a suturing ring to the annular body to facilitate sewing or suturing of the heart valve to the heart tissue. The suturing member may have a rigid annular member or sleeve surrounding the base. The sleeve may be of a rigid material, such as metal, plastic or alike. The sleeve may have collars of fabric, such as Teflon or Dacron (RE31,400). The valve may have further members, such as a cushioning member and a schock-absorbing member. Examples of mechanical heart valves are described in 3,546,711, 4,011,601, 4,425,670, 3,824,629, 4,725,275, 4,078,268, 4,159,543, 4,535,484, 4,692,165, 5,035,709, 5,037,434.

Xenografts, allografts or autografts are tissue valves. When an autologous graft is used, usually the pulmonary valve is operated to the aortic position - a Ross operation. Allografts, also called homografts, are of cadaveric origin. Xenograft bioprosthetic heart valves are usually of porcine origin. They can be stented or stentless. The traditional stented valves may be designed to have a valving element, stent assembly and a suture ring. The stent may be cloth covered. All the known stent materials can be used in the stent, including but not limited to titanium, Delrin, polyacetal, polypropylene, and Elgiloy. As is known by a person skilled in the art, there are several ways to manipulate tissue valves. For example a bioprosthesis may be made acellular (Wilson, Ann Thorac Surg, 1995;60 (2 suppl):S353-8) or preserved in various ways, such as with glutaraldehyde, glycerol (Hoffman), dye-mediated photooxidation (Schoen, J Heart Valve Dis, 1998; 7(2):174-9), and if preserved with glutaraldehyde, glutaraldehyde can be neutralised by aminoreagents (e.g. 4405327). Homografts can be deendothelialised. Examples of tissue heart valves are described in 3,755,823, 4,441,216, 4,172,295, 4,192,020, 4,106,129, 4,501,030, 4,648,881. Also, there exists an extensive scientific litterature in the subject. It will be understood by one skillfull in the art, that any material or tissue with biocompatibility to allow endothelial growth will be acceptable. Genes can be attached to the heart valve prostheses by various methods but the method is not important as long as gene is taken up by the surrounding tissue and angiogenic factors are produced and angiogenesis is stimulated, which results in endothelialisation of the orifice ring and/or the valving member surface. Nucleic acids or a composition comprising nucleic acids may be attached to whole or parts of the heart valves. Preferably, in tissue valves nucleic acids will be attached to the whole surface and stent assembly, and in mechanical valves to annular body and sewing ring.

Tissue implants can be made of various materials, such as polyethylene, polypropylene, polytetrafluorethylene (PTFE), cellulose acetate, cellulose nitrate, polycarbonate, polyester, nylon, polysulfone, mixed esters of cellulose, polyvinylidene difluoride, silicone, collagen and polyacrylonitrile. Preferred support materials for tissue engineering are synthetic polymers, including oligomers, homopolymers, and copolymers resulting from either addition or condensation polymerisations. Examples of tissue implants are described in e.g. 5,314,471, 5,882,354, 5,874,099, 5,776,747, 5,855,613. It will be understood by one skillfull in the art that any material with biocompatibility to allow endothelial growth and/or capillarisation will be acceptable. Genes can be attached to the implant by various methods, but the method is not important as long as gene is taken up by the surrounding tissue and angiogenic factors are produced and angiogenesis is stimulated resulting in endothelialisation and/or capillarisation of the implant.

Background for anastomotic devices also called graft connectors is well described in 5,904,697 and 5,868,763. Generally, anastomotic devices are employed either in end-to-end anastomosis or end-to-side anastomosis. This invention comprises end-to-side anastomotic devices, preferably those anastomotic devices that have an anchoring member being implanted intraluminally to the target vessel and exposed to blood, such as SOLEM Graft-Connector™. The term "anchoring member" is here referred to the member forming the attachment with the target vessel. The term."coupling member" or "connecting member" here refers to the member that forms attachment with the bypass graft vessel. Anchoring member and coupling member may form one single unit or be separated being connected during the procedure. Additional members such as a handle and pins may be comprised. The intraluminal anchoring member may be of various design, preferably it is a tubular structure. The intraluminal anchoring member may be made of any biocompatible material such as metal, ceramic, plastic, polymer, PTFE, DACRON, PET, polypropylen, polyethylen, polyurethane, HOPE, silicone, polyolefins and ePTFE or combination of several structures. Also a biodegradable or bioabsorbable material such as homopolymers e.g. poly-paradioxanone, polylysine or polyglycolic acid and copolymers e.g. polylactic acid and polyglycolic acids or other bio materials may be used either alone or in combination with other materials. Anastomotic device may be porous, partly porous, or nonporous. Preferably the connecting member is nonporous and the anchoring member is porous. Alternatively both the connecting member and anchoring member are porous. If porous, the cross sectional dimensions of the pore capillary diameter are greater than 5 microns and typically less than 1 mm. Upper pore size value is not critical so long as the biomaterial retains sufficient rigidity, however it is unlikely that useful devices would have pore size greater than about 1mm. Such pore dimensions can be quantified in microscope. Suitable pores can exist in the form of channels or passages starting at the external surface and extend through the biomaterial. As will be understood by those in the art several modifications of graft connector design materials and surfaces can be made such as precoating with proteins, non-heparinised whole blood and platelet rich plasma, glow-discharge modifications of surfaces, adding pluronic gel, fibrin glue, adhesion molecules, covalent bonding, influencing surface charges with for example carbon (5,827,327, 4,164,045), treating with a surfactant or cleaning agent, mechanically changing the surface characteristics, such as adding grooves and changing the end angles without excluding any other method. Also the implant can be constructed as a hybrid of different internodal distances in inner and outer surface, such as outer 60 microns and inner 20 microns in internodal distance (such as HYBRID PTFE). Even more layers with different internodal distances may be used. They all are intended to fall in the scope of present invention when not inhibiting endothelialisation. Genes can be attached to the implant by various methods, but the method is not important as long as the gene is taken up by the surrounding tissue and angiogenic factors are produced and angiogenesis is stimulated resulting in endothelialisation and/or capillarisation of the implant. Appropriate graft connectors will both deliver the gene composition and also provide a surface for new endothelium growth, i.e., will act as an *in* situ scaffolding through which endothelial cells may migrate. It will be understood by a person skillfull in the art that any material with biocompatibility, rigidity and porosity to allow transgraft growth will be acceptable.

Pacemaker wires are well known in the art and they may be either porous (4,936,317) or nonporous. Pacemaker wires are usually made of metal or metal alloyes, such as cobolt alloys or titanium. It will be understood by one skillfull in the art that any material with biocompatibility to allow endothelial growth will be acceptable. Genes can be attached to the pacemaker wires by any method. After gene is taken up by the surrounding tissue angiogenic factors are produced and angiogenesis is stimulated resulting in endothelialisation of the implant.

Vascular catheters are well known in the art. It will be understood by one skillfull in the art that any material with biocompatibility to allow endothelial growth will be acceptable. Genes may be attached to the vascular catheter by any method included in this disclosure. After gene is taken up by the surrounding tissue angiogenic factors are produced and angiogenesis is stimulated resulting in endothelialisation of the implant.

Suture materials are well known in the art. "Filament" is here referred a single, long, thin flexible structure of a non-absorbable or absorbable material. It may be continuos or staple. "Absorbable" filament is here referred one which is absorbed, that is digested or dissolved, in mammalian tissue. Sutures may be monofilament i.e. single filament strands or multifilament i.e. several strands in a braided, twisted or other multifilament construction. and are made of wide variety of materials both natural, such as metal, silk, linen, cotton and catgut ,and synthetic, such as nylon, polypropylene, polyester, polyethylene, polyurethane, polylactide, polyglycolide, copolymers of lactide and glycolide. Sutures may be porous (4,905,367, 4,281,669) or nonporous and they can be coated with various materials described in for example in 4,185,637, 4,649,920, 4,201,216, 4,983,180, 4,711,241 or uncoated. Nucleic acids may be attached to any suture material to promote endothelialisation of sutures. Attachment of the nucleic acids is particularly useful with synthetic nonabsorbable vascular sutures. If multifilament suture is to be coated, it is not necessary that every filament within the suture be individually or completely coated. Sizes of suture materials usually range between 12-0 U.S.P. size 0,001 mm to size 2 U.S.P. with outer diameter 0,599 mm. Suture materials may be with or without needle in one or both ends and needle may be attached to the suture material by any of the methods known in the art, such as by defining a blind hole, i.e. a cylindrical recess, extending from a proximal end face of the suture needle along the axis thereof. The length of the suture-mounting portion is generally equal to or slightly greater than the length of the hole. A suture is inserted into the hole and then the suture-mounting portion is crimped, i.e. deformed or compressed, to hold the suture. Alternatively, the suture may be secured by addition of cement material to such blind hole (for example 1,558,037). Also adhesive and bonding agents may be used, such as 2,928,395, 3,394,704. Also other modifications may be employed such as 4,910,377, 4,901,722, 4,890,614, 4,805,292, 5,102,418. The surgical needle itself may be made of various materials, such as medically acceptable stainless steel to required diameter. The suture attachment to the needle may be standard i.e. the suture is securely attached and is not intended to be separable therefrom, except by cutting or severing the suture ,or detachable or removable i.e. be separable in response to a force exerted by the surgeon (3,890,975, 3,980,177, 5,102,418). Surgical needles may be of various form such as ¼ circle, 3/8 circle, ½ curve, ½ circle, 5/8 circle, or straight and the needle distal point may be taper point, taper cut, reverse cutting, precision point, spatula-type, and the like. The amount of nucleic acid attached to the suture material or to the composition coating the suture will vary depending upon the construction of the fiber, e.g. the number of filaments and tightness of braid or twist and the coposition of the composition, solid or solution applied. It will be understood by one skillfull in the art that any material with biocompatibility to allow angiogenesis will be acceptable. Genes can be attached to the sutures by any of the methods described in this disclosure or any other method if so preferred. After gene is taken up by the surrounding tissue angiogenic factors are produced and angiogenesis is stimulated resulting in endothelialisation of the suture material surface.

Surgical pledgets are well known in the art. It will be understood by one skillfull in the art that any material with biocompatibility to allow endothelial growth will be acceptable. Genes can be attached to the surgical pledgets by any method included in this disclosure, or any other method. After gene is taken up by the surrounding tissue angiogenic factors are produced and angiogenesis is stimulated resulting in endothelialisation of the implant.

Physical and chemical characteristics, such as e.g. biocompatibility, biodegradability, strength, rigidity, porosity, interface properties, durability and even cosmetic appearance may be considered in choosing the said vascular or tissue implant, as is well known for those skilled in the art. Also, an important aspect of the present invention is its use in connection with other implants having the advantage of vascularisation of the interface with the tissues, including implants themselves and functional parts of the implant, such as tissue chambers, pacemaker wires, indwelling vascular catheters for long time use and the like. The surface may be coated of pores filled with a material having an affinity for nucleic acids, and then the coated-surface may be further coated with the gene or nucleic acid that one wishes to transfer. The available chemical groups of the adsorptive, may be readily manipulated to control its affinity for nucleic acids, as is known to those skilled in the art.

### METHODS - CONSTRUCTION OF EXPRESSION PLASMIDS FOR VEGF165, FGF-2 AND FGF-5 AND FUNCTIONAL TESTING OF EXPRESSED PROTEINS.

### Expression plasmids.

### VEGF165 expression plasmid.

The human VEGF₁₆₅ cDNA (Medline accession # M32977) was PCR amplified with template cDNA prepared from total RNA isolated from HEK293 cells. In order to increase VEGF₁₆₅ mRNA transcripts, HEK293 cells were treated with 130 µM of deferoxamine for 24 h to mimic a hypoxic environment. Total RNA was prepared using the Trizol® reagent (Gibco BRL) according to the manufacturers instructions. Integrity of purified RNA was analyzed by 1% agarose gel electrophoresis. Complementary DNA was synthesized by reverse transcription using the Advantage® RT-for-PCR kit (Clontech) with oligo dT primers.
The VEGF₁₆₅ cDNA was PCR amplified using the sense primer GATCGAATTCGTTA-ACCA-TGAACTTTCTGCTGTCTTGG containing an *Eco R1* site and the antisense primer GATCGGATCCGTTAACTCACCGCCTCGGCTTGTCACATC with an engineered *Bam H1* site. Amplification was performed with Platinum® *Taq* DNA polymerase (Gibco BRL) according to the manufacturers instructions with the following cycling parameters: 94°C for 1 min and then 35 cycles of 94°C, 30 s; 60°C, 30 s; 72°C, 1 min. The PCR reaction mixture was electrophoresed in a 2% low gelling temperature agarose gel and amplified cDNA visualized by ethidium bromide staining. A product with the expected size of 606 nucleotides was cut of the gel and purified (QIAquick™ gel extraction kit, Qiagen).
The purified product, and the expression vector pNGVL3, was digested with a combination of *Eco R1* and *Bam H1*. The VEGF₁₆₅ cDNA was then directionally ligated into pNGVL3 (Ligation Express™ Kit, Clontech) followed by transformation of chemically competent DH5α *E. Coli* and bacterial colonies selected on LB agar plates containing 30 µg/ml of kanamycine. Single bacterial colonies were isolated, grown in 3 ml liquid cultures and plasmid DNA purified (QIAprep spin miniprep kit, Qiagen). The identity and correctness of the final pNGVL3-VEGF₁₆₅ construct was verified by DNA sequencing using an ABI automated sequencer.

### FGF-2 expression plasmid.

Human FGF-2 cDNA (Genbank accession # NJ04513) was PCR amplified with template cDNA from HEK293 cells prepared as described above for VEGF₁₆₅ expression plasmid. PCR amplification was performed with the sense primer ATACTCTAGA-ATGGCAGCCGGGAGCATCACCACGCTG, containing an *Xba1* restriction site, in combination with the antisense primer GATCAGATCTTCAGCTCTTAGCAGA-CATTGGAAGAAA containing *a BglII* restriction site. Amplification parameters were as described above. The resulting product, with the expected size of 488 nucleotides, was cut out of the gel and purified (as described above) whereafter it was digested with *Xba1* and *BglII*. The restriction enzyme digested product was directionally ligated into the expression vector pNGVL7 (that had been digested with *Xba1* and *BamH1*), bacterial colonies isolated and the identity and correctness of the resulting pNGVL7-FGF-2 construct verified by automated DNA sequencing.

### FGF-5 expression plasmid.

Human FGF-5 cDNA (Genbank accession # M 37825) was PCR amplified using the sense primer GATCGAATTCGTTAACGCCACCGAGCTTGTCCTTCCTCCTCCTC, with an *EcoR1* site, in combination with the antisense primer GATCTCTAGAGTTA-ACTTATCCAAAGCGAAACTTG-AGTCT with an *Xba1* site. The GeneStorm® expression-ready clone H-NM_004464 (Invitrogen) was used as a template. PCR cycling parameters were: 94°C for 1 min and then 30 cycles of 94°C, 30 s; 65°C, 30 s; 72°C, 1 min. The resulting product of expected 842 nucleotides was gel purified, digested with *EcoR1* and *Xba1* and ligated into the corresponding restriction sites in the expression vector pNGVL-3. After transformation of chemically competent DH5α *E. Coli,* a single bacterial colony was isolated and DNA purified. Automated DNA sequencing of the resulting pNGVL3-FGF-5 plasmid was performed to confirm sequence identity and correct orientation.

### β-galactosidase control plasmid.

The pNGVL1-nt-β-gal expression plasmid encodes nuclear targeted β-galactosidase and is used as a control plasmid.

### Cell culture, transient transfections and production of VEGF₁₆₅, FGF-2 and FGF-5.

HEK293T cells were cultured in Dulbecco's Modified Eagle Medium (DMEM; Gibco BRL) supplemented with 10% FBS (Gibco BRL) on gelatin coated tissue culture plastics. Cells were seeded onto 10 cm culture dishes (6 x 10⁶ cells/dish) 24 hours before transfection. Plasmid DNA (4 µg) was mixed with 0.5 ml 2.5 M Ca₂PO₄ and added dropwise to 0.5 ml 2X Hepes buffered saline (HeBSS; 280mM NaCl, 50 mM Hepes, 1.5 mM Na₂HPO₄), vortexed briefly and incubated at room temperature for 20 minutes. The DNA solution was added dropwise to cells and cells incubated with the DNA for 4h after which the cells were treated with 10% glycerol in DMEM for 2 minutes, washed with PBS and fed complete media. 24 h after transfection media was removed, cells washed twice with 5 ml PBS wherafter 5 ml of serumfree DMEM (without supplements) was added. After an additional incubation of 24 h this media was recovered as "conditioned media" and frozen in aliquots for subsequent analysis.

### Western blotting.

An aliquot (50 µl) of conditioned media was mixed with Laemmli sample buffer and run on a 12% SDS/PAGE gel to separate proteins. When dimerization of VEGF was investigated, no reducing agent was included in the Laemmli sample buffer. Proteins were then transferred to Hybond™-C extra membranes (Amersham Life Science) by semi-dry blotting. Nonspecific binding was blocked by incubating membranes in Tris buffered saline (TBS; 20 mM Tris base pH 7.6, 137 mM NaCl) with 0.1% Tween® 20 and 5% BSA (TBS/Tween/BSA) at room temperature for 1h, wherafter filters were incubated for 1 h with specific primary antibodies (diluted 1:500 in TBS/Tween/BSA). VEGF₁₆₅ was visualized with a rabbit polyclonal antibody (Santa Cruz, cat. no. SC-152), FGF-2 with a goat polyclonal antibody (Santa Cruz, cat. no. sc-79-G) and FGF-5 with a polyclonal goat antibody (Santa Cruz, cat. No. sc-1363). Finally, membranes were incubated with horse radish peroxidase (HRP) conjugated secondary antibodies (Anti-goat HRP from Sigma cat. no. A4174 and anti-rabbit HRP cat. no. NA934 from Amersham Life Science at 1:5000 dilution in TBS/Tween/BSA) for 1h and proteins visualized by exposure to medical X-ray films (Fuji) after addition of a ECL western blotting detection reagent (Amersham Pharmacia Biotech).

### Chorioallantoic Membrane Angiogenesis Assay

Fertilized chick eggs were purchased locally and preincubated for ten days at 38 °C at 70% humidity. A 1 cm2 window in the shell exposed the CAM, and an avascular zone was identified for sample application. Whatman filter disks (5 mm in diameter) were saturated with 3 mg/ml cortisone acetate (Sigma) and soaked in conditioned media (containing VEGF₁₆₅, FGF-2 or FGF-5) from transiently transfected HEK293T cells. The window was sealed with tape and incubated for three additional days. The CAM was then cut around the filter and examined using a Nikon Eclipse TE 300 light microscope (magnification 2.5 or 4). Angiogenesis was scored in a double blind procedure for each embryo by estimating the number of vessel branch points in the membrane on the filter disc. The scores ranged from 1 (low, background) to 4 (high). Each substance was analyzed in parallel with 5 to 7 embryos. Sample variation was less than 15%. P values were calculated with ANOVA (analysis ofvariance).

### Preparation of endotoxin-free plasmid DNA for in vivo uses.

Plasmid DNA used for *in vivo* peroperative application was purified with the EndoFree™ Plasmid Mega Kit (Qiagen) according to instructions supplied by the manufacturer and finally resuspended in endotoxin free TE (10 mM Tris pH 8.0, 1 mM EDTA). The quality of the resulting plasmid preparations was checked by spectrophotometric analysis where the A₂₆₀/A₂₈₀ ratio was between 1.80 and 1.82. Also, preparations were checked by restriction enzyme digests with appropriate restriction enzymes and by transient transfections of HEK293 cells followed by Western blotting of conditioned media.

### RT-PCR

Total RNA vas purified from snap frozen tissues using the Trizol® reagent (Gibco BRL) according to the manufacturers instructions. Integrity of purified RNA was analyzed by 1% agarose gel electrophoresis. Complementary DNA was synthesized by reverse transcription using the Advantage® RT-for-PCR kit (Clontech) with oligo dT primers. PCR was performed using the vector derived sense primer CGCGCGCGCCACCAGACATAATAGCTG based on vector sequence 111 bp upstream of the multiple cloning site and the human specific VEGF₁₆₅ antisense primer GCAAGTACGTTCGTTTAACTCAAGCTG 21 bp from the carboxyterminal end of the human VEGF sequence. Amplification parameters were: 94°C for 1 min and then 35 cycles of 94°C, 30 s; 60°C, 30 s; 72°C, 1 min. Amplified products were visualized by UV light after separation on a 2% low melting temperature agarose gel and staining with ethidium bromide. Primers suitable for amplification of GAPDH were used as positive control. cDNA synthesis without addition of RT served as negative controls.

### RESULTS

### Plasmid construction and expression of VEGF₁₆₅, FGF-2 and FGF-5.

We used the pNGVL family of expression plasmids to express the different growth factors. These vectors have been developed for gene therapy purposes at the National Gene Vector Laboratories, The University of Michigan Medical Center, Center for Gene Therapy. VEGF₁₆₅ and FGF-5 contain signal sequences that direct them for secretion. Therefore, the entire coding sequences for these genes were PCR cloned and directly cloned into the expression plasmid pNGVL3. FGF-2 lacks signal sequence and, therefore, the coding sequence for human FGF-2 was cloned into the expression pNGVL7 in frame with the signal sequence for tissue type plasminogen activator (tPA; provided in the pNGVL7 plasmid) to allow the secretion of FGF-2. The inserts, and vector sequences in close proximity to the inserts, were subjected to DNA sequencing to verified that intended cDNA had been amplified, without errors, and ligated into the expression vector in correct orientation.
Transient transfection of HEK293 cells was used to show that the plasmids were able to express the intended proteins. After transfection, serum free conditioned media was collected between 24-48 h post-transfection. Aliquots of conditioned media were analyzed by western blotting using specific antibodies (Fig 1). Conditioned media from cells transfected with empty vectors served as negative control. VEGF₁₆₅ appeared at the expected size of approximately 21 kDa. In addition there was a larger protein detected (23kDa) that probably reflects glycosylated VEGF₁₆₅. Also, when non-reducing conditions were used, the expected dimeric form of VEGF₁₆₅ was observed. FGF-2 and FGF-5 appeared as distinct bands at the expected size of 17 and 27 kDa respectively.

### Functional testing of VEGF₁₆₅, FGF-2 and FGF-5.

The before mentioned growth factors are known to be mitogenic, chemotactic and induce differentiation of endothelial cells. All these processes are necessary for formation of new blood vessels, angiogenesis, and it has been previously shown that all of them induce angiogenesis. Therefore, to further characterize these secreted recombinant growth factors, we employed an *in vivo* angiogenesis assay, the so-called chorioallantoic chick membrane (CAM) assay. Application of media conditioned with VEGF₁₆₅, FGF-2 or FGF-5 all induced an increased angiogenic response in the CAM assay compared to conditioned media from cells transfected with empty vector (Fig 2). These experiments showed that the factors produced by the expression plasmids described above were biologically active since they were able to stimulate the complex process of angiogenesis.

### RT-PCR to prove gene transfer in vivo.

RT-PCR was used to further prove that plasmid DNA's encoding VEGF₁₆₅, FGF-2 and FGF-5 were transcribed after application to tissues *in vivo*. Plasmid DNA was applied around abdominal aortas and the tissue excised 7 days later and snap frozen in liquid nitrogen. Total RNA was prepared from excised tissues and then reverse transcribed using oligo dT priming. For negative controls, the RT was omitted from the reaction mixture. PCR amplified a fragment with the correct expected size of 666 bp when pNGVL-165 was used while RT-PCR of tissues treated with the pNGVL-β-gal plasmid did not give any PCR product (Fig 3). No amplification product was observed in negative control samples (Fig 3). Amplification using GAPDH primers resulted in a product of the expected size.

### Example 1.

### ePTFE graft in rats

This example demonstrates that administration of VEGF plasmid in sterile water solution results in endothelial surface on an ePTFE graft

### METHODS

### Genetic methods:

Construction and evaluation of expression of plasmid encoding for VEGF 165 was performed according to previous methods. VEGF plasmids were given in sterile water at a concentration 2 µg/µL

### Surgical methods:

Four male Sprague Dawley rats were devided in four groups to study endothelialisation of synthetic ePTFE grafts. 3 rats underwent replacement of infrarenal aorta and transfection with an expression plasmid encoding h-VEGF 165. The amounts of the expression plasmid used were 200 µg (n=1), 400 µg (n=1) and 800 µg (n=1) respectively. One rat received a graft without gene transfer. The effect of VEGF transfection was analyzed by histology and scanning electron microscopy (SEM) 2 weeks after transfection.
Anaestesia was induced by a 1 ml intraperitoneal injection of a mixture containing 1,25 mg/ml midazolam, 2,5 mg/ml fluanisone and 0,079 mg/ml fentanyl citrate. In addition, dihydrostreptomycin 25 mg/kg and bensylpenicillinprokain 20 mg/kg was given i.m. Rat was placed on its back, sterile draped, Klorhexidin cleaned and shaved with a machine. Animal was placed on a heating pad and abdomen was sprayed with 70 % Ethanol. Incision was made and aorta was dissected free from vena cava. It was exposed from renal arteries to bifurcation. Part of the aorta including exit of lumbar branches was tied proximally and distally. Aorta was clamped proximally and distally and aortotomy was performed between clamps and ties to accommodate the 2 cm graft.. Aortic stumps were flushed with saline, gently dilated with forceps and aortic graft (20 mm x 2 mm) sutured end-to-end with 9-0 nonresorbable monofilament sutures. The used graft was a porous ePTFE graft with internodal distance of 60 microns (Impra, Tucson, USA) and manufactured according to ILN 150 pp.44-46. Gene solution was administered onto the graft with a pipette. After waiting 3 minutes, abdomen was closed in layers first fascia with running 3-0 and then skin with running 3-0. Starch free gloves were used during surgery. Animal was moved to the recovery area with a warming pad.

### Sacrifice:

Animals were sacrificed at day 14 (n=4) and aortic graft with attached aorta harvested. After anesthesia with fentanyl/fluanisone and midazolam as above and 500 units of heparin in penile vein abdominal aorta was exposed and sternotomy performed. PBS perfusion at 120 mmHg was given to left ventricle through a wide bore needle while the animal was synchronously exsanguinated through incision in the right atrium. Once blood was cleared, the animal was perfusion fixed in situ for 10 minutes at 120 mmHg with 2% paraformaldehyde and 0,2% glutaraldehyde in PBS. Graft was dissected free and excised with 1-2 cm of native vessels and surrounding fat tissue. It was cut longitudinally and divided in five parts. Part A was preserved in 2% paraformaldehyde/0,2% glutaraldehyd for histology, part B in 4% formaldehyde for light microscopy and immunohistochemistry, part C in 2% paraformaldehyde/2 % glutaraldehyd in PBS for SEM, part D in 2% paraformaldehyde/0,2% glutaraldehyd for histology and part E in 4% formaldehyde for light microscopy and immunohistochemistry.

### Graft analysis:

Part C of grafts were analysed with SEM to identify cellularity of the surface and to ensure that the cellularity originated from transgraft growth. Light microscopy was performed of Part B after immunohistochemical staining to measure endothelial cell coverage of the inner surface of the graft and to estimate development of new capillaries.

### RESULTS

### Endothelialisation and vascularisation

Light microscopy performed after immunostaining with factor VIII antibody showed increased staining for FVIII in the tissue surrounding the VEGF treated grafts compared to control graft. Also, near complete endothelialization of the graft inner lumen was noticed among the VEGF treated grafts whereas no endothelialization of control grafts was observed. There was a dose dependent increase in FVIII staining of the surrounding tissue. In control graft endothelium was lacking in the graft inner lumen. At least partial endothelialisation was demonstrated in all VEGF treated grafts.
SEM disclosed that 67% of VEGF treated arteries had endothelial cell coverage in the part C whereas in control grafts no endothelial cell coverage could be shown.

### Gene expression

Gene expression was assessed by in situ hybridisation and demonstrated no expresssion of human VEGF in control graft while strong expression was seen in luminal endothelial cells in two of the three VEGF treated grafts and in one treted graft weak expression was observed.

### Example 2

### ePTFE graft in rats

This example demonstrates that administration of VEGF plasmid in sterile water solution gives faster and more complete endothelialisation of an ePTFE graft

### METHODS

### Genetic methods

Genetic methods were similar to the description in the former example. Plasmids were given in sterile water. The amounts were; LacZ 100 µg (2,5 µg/mL or 2,7 µg/µL), VEGF 100 µg (2 µg/µL or 2,7 µg/µL), VEGF 400 µg (2 µg/µL or 2,4 µg/µL) and VEGF 800 µg (2,3 µg/µL).

### Surgical methods:

37 rats were divided in four groups to study endothelialisation of synthetic vascular grafts. 23 rats underwent replacement of infrarenal aorta and transfection with an expression plasmid encoding h-VEGF 165. Amounts of plasmid used were; 100 µg (n=7), 400 µg (n=12) and 800 µg h-VEGF 165 (n=4). 14 rats were used as controls and received graft with either 100 µg of β-gal (LacZ) (n=9) or or no gene trasfer (n=5). Analysis of grafts and surrounding tissue with histology and SEM was performed at 1 week (day 7-8) (n=7), 2 weeks (days 14-15) (n=17) and 4 weeks (days 28-31) (n=13). Animal was prepared for surgery and operated as described in example 1.

### Sacrifice:

Sacrifice procedure was performed as described in example 1. The graft was cut longitudinally, photographed, and divided into two parts. Then the distal half was further divided into two parts. Part A was preserved in 2% paraformaldehyde/2% glutaraldehyd for SEM, part B in 4% formaldehyde for light microscopy and immunohistochemistry and part C in 2% formaldehyde/0,2 % glutaraldehyd in PBS for light microscopy.

### Graft analysis:

Planimetric analysis, SEM and immunohistochemistry was used to determine endothelialisation of the graft luminal surface. Electron microscopy could distinguish between longitudinal cell migration from the anastomosis and transmural migration.

### RESULTS

### Endothelialisation

Planimetric analysis performed with Evans blue showed 89 % endothelialial coverage among the VEGF treated grafts at four weeks whereas only an average of 44 % of the luminal surface was endothelialized in control grafts at four weeks.
SEM disclosed similar findings. At one week none of the grafts had endothelial coverage of the midgraft area. At 2 weeks 55 % of the VEGF treated grafts were covered by endothelial surface in the midgraft area whereas only 17% of the control grafts were covered in the midgraft area by endothelium. Similarly, at four weeks 88 % of the VEGF treated grafts were endothelialized and 17 % of the controls showed endothelial surface in the midgraft area. Also, transgraft growth was visualised in the midgraft area in VEGF group.
Light microscopy after immunostaining of part B showed complete endothelialisation in 50% of the VEGF treated grafts (400 µg) at four weeks whereas the endothelialization remained incomplete in all control grafts at four weeks. Also, all the VEGF treated grafts had endothelial surface greater than half of the luminal surface. Totally, 25% of the VEGF treated grafts showed complete endothelialisation at four weeks, wherease none of the control grafts showed complete endothelialization at this time point. Endothelialisation was VEGF dose dependent. None of control grafts showed complete endothelialization and only 40% of the control grafts showed endothelial coverage greater than half of the surface at four weeks.

### Example 3

### ePTFE graft in rats

This example demonstrates that simultaneous administration of naked FGF-2 and FGF-5 plasmid gives faster endothelialisation of an ePTFE graft

### METHODS

### Genetic methods

Methods as described before were used for FGF-2 and FGF-5. 500 µg of FGF-2 expression plasmid and 500 µg of FGF-5 expressionplasmid were given in sterile water at concentration 2 µg/µL. FGF-2 was administered with a pipette first and then FGF-5.

### Surgical methods:

6 male Sprague Dawley rats were devided in three time points and compared to same controls as used in example 2 to study endothelialisation of same ePTFE grafts. Number of controls was n=3 at 1 week, n=6 at 2 weeks and n=5 at 4 weeks. 6 rats underwent a replacement of infrarenal aorta and cotransfection with FGF-2&FGF-5 with doses 500 µg&500 µg, respectively. The histologic consequencies FGF-2&FGF-5 transfection were studied at 1 week (n=2), 2 weeks (n=3) and 4 weeks (n=1). Surgery, sacrifice, graft preservation and analysis were performed as described in examples before.

### RESULTS

### Endothelialisation

Planimetric analysis performed with Evans blue showed 92 % endothelialial coverage in the FGF treated graft at four weeks whereas the endothelialization in control grafts was in average 44 % of the surface area at four weeks.
SEM of part A disclosed complete endothelialisation in midgraft area among the FGF treated grafts at one week whereas none of the control grafts had a complete endothelial surface in the midgraft area at the same timepoint. In FGF treated grafts endothelialisation of the midgraft area was 100 % at two weeks whereas 17% of the control grafts showed endothelialization of midgraft area at 2 weeks. Similarly, at four weeks 100 % of the FGF treated graft was endothelialized and 20 % of the controls showed endothelial surface in the midgraft area. Transgraft growth was visualised in FGF treated grafts at one, two and four weeks.
Light microscopy after immunostaining of part B showed at one week over 50 % endothelialisation in one and complete endothelialisation in the other FGF treated graft and at two weeks 67% of FGF grafts had more than 50% of the surface covered whereas none of the control grafts had more than 50 % covered of the graft inner surface at 14 days. Also, all control grafts remained incompletely endothelialised at four weeks whereas FGF treated graft was completely endothelialised at four weeks.

### Example 4

### ePTFE graft with fibrin glue in rats

This example demonstrates that administration of VEGF plasmid in fibrin glue gives endothelial surface on a ePTFE graft

### METHODS

### Genetic methods

Genetic methods were similar as described above.

### Surgical methods:

Four rats were devided in two groups to study endothelialisation of synthetic ePTFE graft. Rats underwent a replacement of infrarenal aorta according to previous examples and transfection with h-VEGF 165 given from fibringlue. Two rats received graft and glue with sterile water without the plasmid. The histologic consequencies VEGF transfection were studied at 2 weeks. The surgical procedure was performed as described in examples before. After anastomosing the graft the glue was administered through with a duploject (Duo Mix) on the graft. 0,6 mL of VEGF plasmid in sterile water (2 µg/µL) was injected to a syringe containing commercially available 0,4 mL human thrombin (Thrombin, Immuno, Austria). Then thrombin and plasmid combination was drawn back and forth between two syringes through a three way stopcok to make an even blend of the two components. After performing the surgical anastomosis 0,1 mL of fibrinogen (Tisseel, Immuno, Austria) and 0,15 mL thrombin-plasmid mixture were administered simultaneously through a Tisseel Duo Mix applicator on the graft. In control graft same amount of sterile water without plasmid was given. Sacrifice and grafts analysis were performed as described before.

### RESULTS

### Endothelialisation.

SEM of part A disclosed that at 1 week 50 % of the VEGF treated grafts were covered by endothelial cells in the midgraft area whereas none of the control grafts showed endothelial cell lining.
Light microscopy after immunostaining of part B showed near complete endothelialisation among 50 % the VEGF grafts whereas none of the grafts in the control group had over 50 % of the surface covered by endothelium at 1 week.

### Example 5

### ePTFE graft with hyaluronic acid-fibrin glue in rats

This example demonstrates that co-administration of VEGF and FGF-2 plasmids in fibrin glue mixed with hyaluronic acid reduces thrombogenicity of an ePTFE graft

### METHODS

### Genetic methods and preparation of the glue.

FGF-2 and VEGF were prepared according to methods described in former examples. Plasmids were given in a glue composition and administered according to the following description. Commercially available human fibrinogen (Tisseel, Immuno, Austria) was warmed to 37 C in water. 0,7 mL of the fibrinogen and 0,7 mL of commercially available hyaluronic acid (Healon GV 14 mg/mL, Kabi Pharmacia) was drawn into two syringes. A three way stopcock was connected to the syringes and fibrinogen and hyaluronic acid were drawn back and forth between the syringes to get an even mixture. Then separately 0,3 mL of VEGF plasmid in sterile water (2 µg/µL), 0,1ml FGF-2 plasmid in sterile water (5 µg/µL) and 0,05 mL heparin (1000 U/mL) were drawn into one syringe and commercially available thrombin (Immuno, Austria) in another syringe. They were connected to the ports of an another stopcock. Thrombin and plasmid combination was drawn back and forth through a 3-way stopcock between the syringes to achieve an even blend of the components. After the surgical anastomosis of the graft was performed the 0,25 mL hyaluronic acid/fibrinogen and 0,25 mL thrombin/plasmid/heparin compositions were administered on the graft through a Tisseel Duo Mix applicator. After the polymerisation of the glue polymerisation and the surgical procedure was completed.

### Surgical methods:

2 male Sprague Dawley rats were devided in two groups to study endothelialisation of same ePTFE grafts as in former examples and underwent a replacement of infrarenal aorta and co-transfection with h-VEGF 165 and human FGF-2 given in hyaluronic acid-heparin-fibrin glue composition. 1 rats received graft and glue with sterile water without the plasmid. The histologic consequencies VEGF and FGF-2 co-transfection were studied at 7 days. The surgical procedure and graft analysis were performed as described in examples before.

### RESULTS

### Endothelialisation

Planimetry demonstrated that glue-FGF-VEGF treated graft was covered at one week by endothelial cells to 46 % whereas control graft was covered to 3 % by endothelium. In the control graft there was thrombus .

SEM of part A disclosed that at 1 weeks VEGF-FGF graft was open and had cellular graft surface whereas in control graft there was major thrombus formation.

### Example 6

### ePTFE graft with bound mucin in rats

In this example VEGF plasmids were bound to the ePTFE graft with mucin and endothelial surface resulted

### METHODS

### Purification of commercial BSM (Sigma M3895)

BSM (Sigma M3895) was purified by sequential use of anionic exchanger (Q Sepharose by Amersham Pharmacia Biotech) and gel filtration (Sepharose 6B-CL by Amersham Pharmacia Biotech).

### Preparation of aminated PTFE.

Expanded PTFE (ePTFE) was plasma treated according to the following: pretreatment was performed with O₂, 8 cc/min, 14 MHz, 100 W for 30 s. Then amination was performed with diaminocyclohexane (DACH), 18 mTorr, 170 kHz, 10 W for 2 min and thereafter graft was refrigerated in desicator until use.

Other needed material for this example was 7,5 mL and 20 mL glass vials. Also, MilliQ and TBS pH 7,4 were used as well as tips and wettex pads cut into 1 x 1 cm. 20 % glucose and sterile filtered PEI stock solution (90 µM) were used. 70 % EtOH was used as a bacteriostat. Target-plasmid (VEGF) and control plasmid (β-gal) were at 2 mg/ml in TE buffer pH 8,0.
Grafts were sterilized in autoclave at 125°C for 25 minutes and then incubated in BSM (bovine saline mucin) fraction QS1A (high MW, high relative charge) at 2 mg/ml in TBS and pH 7,4. Incubation was performed with shaker 200 rpm over night at 37°C. (17:55-9:30, i.e. 15,5 hours)
Then grafts were washed. 10 ml TBS in 50 ml Falcon tubes was used for sequential wash of grafts and wash lines were grouped into "Target" and "Control" wash lines. Tubes were first vigorously shaked and then 1 relaxed one minute. It was repeated twice (2 wash steps). DNA incubation was done after DNA preparation according to protocol described before and system to enhance transfection was made: 2 ml 20 % glucose + 1 ml PEI stock solution + 1 ml plasmid solution + 4 ml MilliQ. Grafts were incubated in each plasmid incubation solution for 2 hours with shaker at 200 rpm at room temperature. Then sequential wash was performed in 10 ml MilliQ in 50 ml Falcon tubes. Wash line was separate for "Target" and "Control" lines. Vigorous shaking and 1 minute of relaxation. This was repeated twice. Thereafter grafts were incubated in 50 ml Falcon tubes with 1 x 1 cm Wettex pad. 0,1 ml MilliQ was added to each tube for moisture. Graft was placed in upper region (cap region) of tube and stored horizontally at 4-8°C until use.
Four rats were divided in two groups to study endothelialisation of porous ePTFE graft. 3 Rats underwent a replacement of infrarenal aorta and transfection with h-VEGF 165 released from the mucin coating of the graft. 1 rat received graft with mucin coating but without plasmid. The histologic and scanning electrone microscopic consequencies of VEGF transfection were studied at 2 weeks.

### RESULTS

### Endothelialisation

SEM of part A disclosed that at 2 weeks 67 % of the VEGF treated grafts were covered by endothelial cells in the midgraft area whereas the control graft had no endothelial surface in the midgraft area.
Light microscopy after immunostaining of part B showed endothelialisation over 50% of the surface area among 67% of the VEGF treated grafts at 14 days whereas no endothelialization was seen in control graft at 14 days.

### Example 7

### Rabbit ePTFE graft

This example demonstrates that administration of naked VEGF plasmid in sterile water on a ePTFE graft results in faster endothelialisation and higher patency rate

### METHODS

New Zealand rabbits (2,5- 4 kg) were used in the experiments in this and following experiments. 9 animals were devided in two groups to study the endothelialisation of the 60 microns internodal ePTFE graft. 5 rabbits underwent a replacement of aorta and transfection with 600 µg h-VEGF 165 and 4 rabbits were used as controls and underwent identical graft replacement with 100 µg B-gal (LacZ) transfection. Histologic and electronmicroscopic consequencies of β-gal/LacZ transfection and VEGF transfection were studied at 2 weeks (n=5) and 12 weeks (n=4).Construction and evaluation of expression of plasmid encoding for VEGF 165 were performed as desribed before. Plasmids were given in sterile water solution with LacZ (2,5 µg/µL;dose 100 µg) and VEGF (2,5 µg/µL;dose 600 µg).

### Surgical arterial reconstruction and gene transfer

320 mg acetylsylic acid was added to drinking water to give an estimated daily ASA dose 10 mg/day. Rabbit was anesthesized with combination of fentanyl 0,315 mg/mL/fluanosine 10 mg/mL mixture s.c. and midazolam 5 mg/ml i.m. Dihydrostreptomycin 25 mg / bensylpenicillinprokain 20 mg/kg was given i.m. and bupivacain 2,5 mg/mL was administered intracutaneously to the wound area. Abdominal midline incision was performed. Aorta was dissected free from vena cava. Last lumbar branch of aorta was identified and ligated. Aorta was exposed proximal to bifurcation and intravenous dextran (Mw 70000 containing 60 g/L dextran in physiologic NaCl) was given over 15 minutes followed by buffered glucose 2,5% 100 mL/hour during the operation. 500 U of heparin was administered i.v. in the ear vein through a venflo. After 4 minutes of heparin circulation aorta was clamped and resected proximal to bifurcation to accommodate the 2 cm long and 3 mm internal diameter ePTFE graft with intemodal distance 60 um (Impra, Tempe, AZ, USA) manufactured according to ILN 150, pp. 44-46. The graft was anastomosed end-to-end with running 7-0 sutures. Retroperitoneum and fascia were closed with 4-0 and skin sutured with 3-0. Thermal barrier heating pads were used postoperatively and rectal temperature was registered until 37 C was reached. Surgical method used in this example was also used in following examples.

### Ex vivo animal examination

Anesthesia was performed as above and 6 mL of 0,5 % Evans blue and 0,2 mL heparin ( 5000 U/mL) were given in the ear vein half an hour before sacrifice. Some animals underwent an MRI examination with use of ketamine in the anesthesia protocol. An abdominal incision and sternotomy were performed. Aorta and heart were exposed. PBS was infused at 120 mmHg pressure through a wide bore needle to the left ventricle while the animal was synchronously exsanguinated via an incision in the right atrium. Once blood was cleared the animal was perfusion fixed in situ for 10 minutes at 120 mmHg with 2% paraformaldehyde/ 0,2% glutaraldehyde in PBS. Graft was dissected free and excised with 1-2 cm of native vessels. The harvested arterial segment was inspected and opened longitudinally. It was photographed for planimetric studies of the thrombus free surface area. Graft was cut into three parts for measurements.

### Analysis of the graft

Planimetric analyses was performed after photographing the harvested graft in dissecting microscope. The area of intimal surface that remained endothelium deficient was stained blue after the application of Evan's blue. The macroscopic analyses were confirmed through immunostaining of light microscopic analysis. Extent of endothelialization was calculated as a percentage of the total intimal area encompassed within the graft. Scanning electron microscopy was performed according to standard methods in proximal half of the graft. SEM pictures were taken in the proximal half in proximal, middle and distal part of the sample. SEM pictures were evaluated from the midgraft area close to the graft middle. SEM pictures were taken to verify transgraft growth. Immunohistochemistry was performed to identify cell types in light microscopy.

### RESULTS

### Endothelialisation

Planimetric analysis performed with Evans blue at two weeks showed 77% endothelialisation among the VEGF treated grafts whereas the endothelialization in LacZ grafts was 27 % of the surface area at 14 days.
SEM disclosed similar findings. At two weeks 67% of VEGF treated grafts had endothelial cells on the surface in the midgraft area whereas none of LacZ grafts were covered by endothelial cells. Also, the transgraft growth could be visualised in the VEGF treated group.
Light microscopy disclosed similar findings. At two weeks 100 % of VEGF treated grafts were verifyid to have endothelial cells on the surface whereas none of LacZ grafts were covered by endothelial cells.
Also, there was difference in patency at three months; patency was macroscopically noticed and histologically verifyid to be 100 % for VEGF treated group and 50 % for the control group.

### Example 8

### Rabbit ePTFE graft

This example demonstrates that co-administration of FGF-2 and FGF-5 plasmid in sterile water on a ePTFE graft results in faster endothelialisation

### METHODS

Two rabbits were transfected with FGF-2 and FGF-5 to study the endothelialisation of the 60 microns internodal ePTFE graft and underwent a replacement of aorta and co-transfection with 500 µg FGF-2 and 500 µg FGF-5. Two LacZ transfected rabbits were used as controls. Histologic and electronmicroscopic consequencies of B-LacZ transfection and FGF-cotransfection were studied 2 weeks (n=4).Construction and evaluation of expression of plasmid encoding for FGF-2 and FGF-5 were performed as desribed in former sections. LacZ (dose 100 µg:2,5 µg/µL), FGF-2 (dose 500 µg:2 µg/µL) and FGF-5 (600 µg:2,5 µg/µL) were given as sterile water solution.

Surgical arterial reconstruction and gene transfer were performed according to previously described methods. First, the dose of FGF-2 was administered on the graft and then FGF-5.

### RESULTS

### Endothelialisation

Planimetric analysis demonstrated that in control grafts endothelial coverage of the surface was in average 27 % at two weeks whereas it was 91% in FGF treated grafts.
SEM disclosed similar findings. At two weeks all of the FGF treated grafts had some endothelialcell coverage of the midgraft area whereas none of LacZ grafts were covered by endothelium in same location. Also, the transgraft growth could be visualised in the FGF group.
Light microscopy disclosed similar findings. At two weeks both of the FGF treated grafts had near complete endothelial cell coverage whereas none of the control grafts showed endothelial lining.

### Example 9

### Rabbit Dacron graft

This example demonstrates that faster endothelialisation of a knitted preclotted Dacron graft results with administration of naked VEGF plasmid in sterile water on the graft.

### METHODS

Five rabbits were divided in two groups to study the endothelialisation of the knitted Dacron graft ( Sulzer Vaskutec). 2 rabbits underwent a replacement of aorta and transfection with 600 µg h-VEGF 165 (n=2). One control rabbit was not transfected and two other ones were transfected with 600 µg B-gal (LacZ). Histologic consequencies of B-LacZ transfection, and VEGF transfection were studied at 1 week (n=2) and 2 weeks (n=3).

### Genetic methods.

Construction and evaluation of expression of plasmid encoding for VEGF 165 was performed according to the methods described before. Plasmids were given in sterile water solution LacZ (600 µg;2,5 µg/µL) and VEGF (600 µg;2,5 µg/µL).

### Surgical arterial reconstruction and ex vivo examination

Procedure was same for the rabbits as described in example 7 except that the 3 cm long and 3 mm internal diameter knitted Dacron graft was used and was preclotted 30 minutes in unheparinised blood from the ear vein. Preclotted graft was manually pressed and the inner lumen cleared. Graft was cut to length of 2 cm and anastomosed to aorta. After sacrifice grafts were analysed with SEM and light microscopy.

### RESULTS

### Endothelialisation

SEM analysis showed smoother surface and endothelial cells in the VEGF graft at one week whereas no endothelialization was noticed at 7 days in the control graft. VEGF treated graft had developed a complete cobblestone surface at 2 weeks whereas 50% of Lac Z treated grafts showed complete surface in SEM at 2 weeks.
Light microscopy showed noncomplete endothelialisation at 7 days in both groups control graft and complete endothelialisation in both groups at two weeks.

### Example 10

### Rabbit Dacron graft

This example demonstrates faster and endothelialisation with FGF-2 and FGF-5 co-transfection of preclotted Dacron.

### METHODS

Six rabbits were divided in two groups to study the endothelialisation of the knitted Dacron graft. Same controls as in example 9 were used. 3 rabbits underwent a replacement of aorta and co-transfection with 500 µg of FGF-2 and 500 µg FGF-5. One untransfected and two LacZ transfected (600 µg) rabbits were used as controls. Histologic consequencies of FGF co-transfection were studied at 1 week (n=2) and 2 weeks (n=4).

### Genetic methods.

Construction of expression of plasmid encoding for FGF-2 and FGF-5 were performed as described before. Plasmids were given in sterile water solution LacZ (600 µg;2,5 µg/µL), FGF-2 (500 µg;2 µg/µL) and FGF-5 (500 µg;2,5 µg/µL). Surgical reconstruction, gene transfer and ex vivo animal examination were performed as described in example 9 except that plasmids were given in sequence; first, FGF-2 was given first around the graft and then FGF-5 was added. Grafts were analysed with SEM and light microscopy as before.

### RESULTS

### Endothelialisation

Scanning electrone microscopy showed partial endothelialisation on the FGF graft at one week whereas no endothelialisation was noticed on control graft. At two weeks one FGF treated graft one beautifull complete endothelialial surface with cobblestone morphology and the other some nonconnected endothelial cells whereas endothelial surface had developed in one of the two control grafts. Light microscopy with immunostaining disclosed that the cells covering the surface were endothelial cells.

### Example 11

### Rabbit ePTFE with fibrin glue

This example demonstrates higher degree of endothelialisation of ePTFE graft treated with VEGF plasmid in fibrin glue.

### METHODS

3 rabbits were divided in two groups to study the endothelialisation of the 60 microns intemodal ePTFE graft. 1 rabbit underwent a replacement of aorta and transfection with h-VEGF 165 in fibrin glue. Another 2 rabbits were used as controls and underwent graft replacement with B-gal (LacZ) transfection in fibrin glue. Histologic and electronmicroscopic consequencies of B-LacZ transfection, and VEGF transfection were studied at 2 weeks (n=3).

Construction and evaluation of expression of plasmid encoding for VEGF 165 were performed as desribed previously and glue was constructed as described hereunder.The histologic consequencies of VEGF and B-gal transfection were studied at 2 weeks. After anastomosing the graft the glue was administered through a Tisseel Duo Mix applicator on the graft. 0,6 mL of VEGF plasmid in sterile water (1200 µg;2 µg/µL) was injected to a syringe containing commercially available 0,4 mL human thrombin (Thrombin, Immuno, Austria). Then, thrombin and plasmid combination was drawn back and forth between two syringes through a three-way-stopcock to make an even blend of the two components. After performing the surgical anastomosis 0,2 mL of fibrinogen (Tisseel, Immuno, Austria) and 0,3 mL thrombin-plasmid mixture were administered simultaneously through a Tisseel Duo Mix applicator on the graft. In control grafts B-gal plasmid was used in sterile water mixed with thrombin.

### RESULTS

### Endothelialisation

Light microscopy disclosed no endothelial cell coverage in the control grafts whereas in VEGF treated graft about half of the surface was covered with endothelial cells. In SEM none of the groups had developed endothelium.

### Example 12

### EPTFE Hybrid graft with fibrin glue

This example demonstrates higher degree of endothelialisation of hybrid graft when VEGF plasmid was administered in fibrin glue.

### METHODS

2 rabbits animals were devided in two groups to study the endothelialisation of the commercially available 60 microns/20 microns internodal distance hybrid graft ePTFE graft (Atrium, New Jersey, USA). 1 rabbit underwent a replacement of aorta and transfection with 600 µg h-VEGF 165. Another 1 rabbit was used as control and underwent identical graft replacement with B-gal (LacZ) transfection. Histologic and SEM consequencies of B-LacZ transfection and VEGF transfection were studied at 2 week (n=2).
Construction and evaluation of expression of plasmid encoding for VEGF 165 was performed according to protocol described before. After 0,4 mL of thrombin had been pushed out from commercially available 1 mL thrombin syringe (Thrombin, Immuno, Austria) 0,6 mL of plasmid solution 2 µg/µL was injected to the syringe containing 0,6 mL human thrombin (Thrombin, Immuno, Austria). Then thrombin and plasmid combination was drawn back and forth between 1 mL syringes (Codan Medical, Denmark) through an 18 G needle (Terumo Europe N.V., 3001 Leuven, Belgium) to make an even blend of the two components. 0,2 mL of fibrinogen (Tisseel, Immuno, Austria) solution was administered around the graft after implantation of the commercially available porous hybrid graft with internodal distance of 60/20 microns (Atrium, New Jersey, USA). 0,2 mL of fibrinogen (Tisseel, Immuno, Austria) solution was administered around the graft. Polymerisation of the fibrinogen locally around the graft was achieved by administering 0,4 mL human thrombin/plasmid combination to produce plasmid containing fibrin glue around the graft.

### RESULTS

### Endothelialisation.

Planimetric analysis performed with Evans blue showed 0,96 % surface coverage with endothelium in the VEGF group, whereas the endothelialization in LacZ grafts was 0,76 % at 14 days.
In SEM higher degree of cellular coverage with endothelial cells was noticed in VEGF group and more transgraft growth could be visualised in the VEGF group.

### EXAMPLE 13

Photooxidized heart valve with or without fibronectin

This example showes faster endothelialisation of heart valve surfaces with or without fibronectin precoating when VEGF plasmid was administered. Also increased capillarisation of the implant was noticed.

### METHODS

VEGF-plasmid was prepared according to procedures described before.
Commercially available photoxidized pericardium (Sulzer Carbomedics, Austin, Texas), used normally as intracardiac patches and in biological heart valves, was removed from the storage solution and rinsed in sterile saline solution (0,9 %) twice for 1 hour. Then the material was left in saline solution for 3 hours. Thereafter pericardium was placed flat and divided in two pieces under sterile conditions.

First half was divided in two pieces. First one was divided in 1 cm² inoperated after administering 0,6 mL sterile water and airdrying for 1 hour. The other half was exposed to plasmid solution (concentration 2 µg/µL;dose 300 µg/cm²) and airdried for one hour.

The second half was precoated with rat fibronectin 0,25 µg/µl (Sigma Chemical Co. St Louis, Mo) at a concentration of 10 µg/cm² and air dried for 1 hour. Thereafter dimeric plasma fibronectin's heparin affinity was utilised by introducing heparin (Löwens, Balle-rup, Denmark) at a concentration 2 U/cm² onto the pericardial surface for 1 hour. Then the fibronectin treated pericardial sheet was divided in two pieces and VEGF- plasmid (2 µg/µL; 300 µg/cm2) was administered on one half and sterile water on the other. Pieces were let to airdry for one hour. Sheets were divided in pieces measuring 1 cm x 1cm and the pieces were inoperated in rats. Control rats received on the right side of the abdominal wall plain control valves and on the left side side control valves with fibronectin and heparin. Treatment animals got on the right side of the abdominal wall valves with the plasmid and on the left side valve with fibronectin/heparin and plasmid. Animals were followed 2 weeks (n=2) and 5 weeks (n=2).
Control 2 weeks: 4 valves on the left side and 4 on the right side
Treatment 2 weeks: 6 valves on the left side and 6 valves onthe right side
Control 5 weeks. 5 valves on the left side and 6 valves on the right side
Treatment 5 weeks; 6 valves on the left side and 6 valves on the right side

### Surgical procedure and sacrifice:

Animals were anesthesized with same anesthesia as used for rats operated with aortic graft. Abdomen was shaved and opened. 2 mL of bupivacain was administered in the wound area. Valves were sewen to the peritoneum on both sides of midline with continuous 5-0 nylon. Each piece was attached separately on the wall with running 5-0 monofilament suture. Abdomen was closed in layers with 3-0. Animals were sacrificed in anesthesia after explanting abdominal wall with heart valve pieces .

### Analysis:

Every piece was divided in the middle. Half of the valve was sent to electron microscopy after preservation in 2% paraformaldehyde/2% glutaraldehyde. Second half was examined in light microscopy after preservation in 4% formalin and immunostained for stained for factor VIII. Two randomly chosen valves from every group were sent to SEM and histology with immunochemistry was investigated in every valve.

### RESULTS

SEM disclosed at two weeks no endothelial cell lining in controls without fibronectin whereas with plasmid trewatment 50% had developed an endothelial surface. In valves treated with only fibronectin 50% had developed an endothelial lining and *with* addition of a plasmid 100% developed an endothelial lining. At four weeks 25% of the control valves had developed an endothelial lining whereas all of the plasmid treated grafts were covered by endothelium with nice cobblestone morphology.
Light microscopy showed at 5 weeks increased capillarisation of the valves in the plasmid treated group compared to the control group.

### Example 14.

### ePTFE stentgrafts in rabbits

This example demonstrates increased endothelial surface and decreased thrombogenisity after VEGF or combined application of VEGF and FGF-2 gene transfer.

### METHODS

Six New Zeland White rabbits (2.5-3.2 kg) were used in the experiments and underwent a bilateral stentgraft placement in the carotid arteries to study the endothelialization.

### Genetic methods:

Same genetic methods were used as described before.

### Construction of stentgraft

The stentgraft was constructed as basically described previously by others (Swedish patent application number 9903674-1). We have used high porosity ePTFE tubing. Initial 2 mm of a 10 cm long polytetrafluoroethylene (PTFE) tubing with 60 µm internodal distance (1.0 mm internal diameter, 0.06 mm wall thickness, Zeus Inc., Orangeburg, SC, U.S.A.) was mounted over a 200 µm pipette tip (Labora, Sweden). A 25 cm long fish string loop (Expert, #340, 0.20 mm thickness, Fladen Fishing) was drawn between the most proximal stent struts (JOSTENT® FLEX, 16 mm long, 3-5 mm post-dilatation diameter, Jomed International AB, Helsingborg, Sweden) until the stent was in the middle of the string. Thereafter, both free string ends were put through the PTFE tubing beginning from the wide end of the pipette tip. The loop with the stent at the tip of the loop was pulled through the pipette tip and PTFE tubing until the proximal end of the stent was emerging from the free end of PTFE tubing. The FIFE tubing was cut at the distal end of the stent. This procedure resulted a stent entirely covered by the PTFE tubing, except for the distal 0.2-0.5 mm ends. The stentgraft was then mounted and crimped on a coronary angioplasty catheter (FREEWAY® PTCA Catheter, 2.0 cm long, 2.5 mm diameter, Jomed International AB, Helsingborg, Sweden) immediately before implatation.

### Carotid artery angioplasty with insertion of stentgrafts

320 mg acetylsalicylic acid (ASA) was added to drinking water to give an estimated daily ASA dose of 10 mg/day. The animals were anesthetized with 0.33 mL/kg subcutaneous Hypnorm® (0.315 mg/mL fentanyl & 10 mg/mL fluanosine, Janssen Pharmaceutica) and 0.33 mL/kg intramuscular Dormicum® (midazolam, 5mg/mL, Roche) and the anesthesia was maintained with intermittent intravenous bolus doses. A combination of 25 mg/kg Dihydrostreptomycin and 20 mg/kg bensylpenicillinprokain was given i.m. and 3 mL marcain (2.5 mg/mL) was administered intracutaneously to the wound area. The neck was shaved and sterile prepped. Under dissecting microscope both common carotid arteries were exposed through a mid-line neck incision. All branches below the bifurcation were ligated with 4-0 Neurolon (Ethicon). 500 IU of intravenous heparin was administered in the marginal vein of the ear. One of the carotid arteries was randomly chosen to be subjected for the intervention. The vessel was occluded proximally and distally with vessel loops and an arteriotomy was made after 4 minutes of after heparin administration to the distal common carotid artery, immediately proximal to the carotid bifurcation. The angioplasty catheter with the stentgraft was guided through the arteriotomy and placed to the proximal common carotid artery. The plasmid solution (600 µg VEGF, or 600µg VEGF with 300µg FGF-2) in 50 µl sterile water, or placebo (50µl sterile water) was drawn to a syringe attached to a tuberculine needle (0.30 mm in diameter) and injected through the vessel wall between the stentgraft and wessel wall at mid-stentgraft position. Immediately after injection the angioplasty catheter with the stentgraft was inflated to 9 ATM for 60 s. Thereafter, the catheter was withdrawn, leaving the stentgraft in place. The arteriotomy was closed surgically with a 10-0 Ethilon suture (Ethicon), vessel loops removed thereby reestablishing the blood flow through the artery. Thereafter, the procedure was repeated on the remaining contralateral carotid artery and the wound closed in layers with 3-0 Monocryl (Ethicon). In these experiments both the left and the right carotid artery of each individual animal received identical treatment.

### Ex vivo animal examination

The animals were anesthetised as above either one week (7 days) or two weeks (14-15 days) after implantation. 6 mL of 0,5 % Evans blue was given in the ear vein half an hour before sacrifice. After bupivacain injection locally a cervical incision and sternotomy were performed. 1000 IU heparin was given intravenously. The aorta and the heart were exposed. Phosphate buffered saline was infused at 120 mmHg through a wide bore needle to the left ventricle while the animal was synchronously exsanguinated through an incision in the right atrium. Once the blood was cleared the perfusion was stopped and the carotid arteries were dissected free. The carotid arteries were explanted and the stented vessel segments were divided transverserly in two halves of equal length. The distal halves were cut open longitudinally, photographed for planimetry and processed further for scanning electron microscopy, as previously described. One of the randomly chosen proximal halves was processed to methylmethacrylate (MMA) inbeddning and further histological examination. The other half was cut open longitudinally, photographed for planimetry and processed further for surface immunohistochemical examination.

Planimetry was performed by two individuals blinded to treatment. Areas of endothelial coverage were determined together by the two investigators to agree consensus.

### SEM

SEM was performed according to the previously described methods. Planimetry on the SEM images was performed by two individuals blinded to treatment. Areas of endothelial coverage were determined together by the two investigators to agree consensus.

### Histological examination

### Methods of histological Analysis

Intact vessel segments containing stentgrafts and 5 mm of adjacent unmanipulated arteries were removed en bloc and immersion fixed in 4% neutral buffered formalin for 12 h. Fixed samples were dehydrated in a graded series of ethanol and infiltrated with a 1:1 solution of MMA and xylene and finally with MMA (4°C, 12 h each). Infiltrated specimens were placed into embedding molds and polymerization was performed at -15°C overnight. Polymerized blocks were initially ground to bring the tissue components closer to the cutting surface.
Two serial sections, five µm thick, 4 mm apart of the same MMA blocks were cut on a Leica 2500 SM sliding microtome with hard tissue blades (Leica, Bensheim, Germany). After immersion in a drop of 80% ethanol sections were stretched to a fold-free state on Superfrost glass slides (Menzel-Gläser, Germany), covered with a polyethylene sheet and several layers of filterpaper, and tightly pressed on the glass slides followed by overnight drying at 42 °C under pressure. Deplastination was carried out in 2-methoxy-ethyl-acetate for 45-90 min. Rehydration of the sections was performed in graded ethanol solutions and 1mM PBS. Hematoxylin and eosin, Masson's trichrome and Elastica van Gieson's stainings were performed according to standard histopathological methods.

### Immunocytochemistry

Sections were heated for 3 min at 90 °C under pressure in 0.1 M citrate buffer for antigen retrieval. Immunohistochemical stainings were performed with the ABC/AEC method. Endogeneous peroxidase was blocked by incubation for 20 min with 0.3% H₂O₂ in methanol, followed by 30 min incubation with Zymed CAS blocking solution (Zymed Laboratories, San Francisco, CA). Sections were then incubated for 1 h with primary antibody, rinsed and secondary antibody was added for 30 min. Avidin-biotin complex was added for 30 min and signal was detected using 3-amino-9-ethyl-carbazole (AEC, Zymed Laboratories). Endothelial cells were detected with polyclonal Ab PECAM-1 (M-20, Santa Cruz Biotechnology, 1:20). Biotinylated secondary antibody was purchased from Dako and used at a dilution of 1:50. Controls for immunostainings included sections incubated with class and species matched irrelevant antibodies and incubations where the primary antibody was omitted.

Histological analysis was performed by an individual blinded to the treatment.

### Surface immunohistochemistry

After over night fixation in 4% phosphate buffered paraformaldehyde, the vessel segment was washed in PBS, and dehydrated in a graded series of ethanol until the final concentration of 50% for storage at +4°C. Before further processing the vessels were rehydrated back to PBS. The specimens were incubated with the primary polyclonal Ab PECAM-1 (M-20, Santa Cruz Biotechnology, 1:250) over night at +4°C, washed with PBS before incubation with secondary Ab (goat anti rabbit IgG, Dako, 1:100) 2h at +4°C, followed by washing in PBS, and incubation with chromogen (3,3'-diaminobenzidine tetrahydrochloride used as the substrate in the peroxidase reaction) 30 min at room temperature. The specimen was then washed in water and the samples observed in a Leica M12 stereo microscope. Images were acquired with a Leica DC100 digital camera.

### RESULTS

### Endothelialisation

Planimetry showed that VEGF plasmid treated stentgraft were covered by endothelial cells to 55.8- 62.0% whereas in control stentgrafts were occluded by thrombosis, i.e. 0 % covered by endothelium, at two weeks. At one week coverage percentages were 57.6 % in VEGF + FGF-2 group, 57.9 % in VEGF group, and 32.9 % in control.

SEM of part A disclosed that at 2 weeks 55.8 - 62.0 % of the stentgrafts treated with the VEGF plasmid were covered by endothelial cells whereas the surface of the control stentgraft was thrombosed, i.e. 0 % covered by the endothelium. At one week VEGF treated stentgraft displayed a 32.7 % coverage with the endothelium, and the VEGF + FGF-2 treated stentgraft 38.9 % coverage. In control stentgraft the endothelial coverage was 21.6%.

Light microscopy after surface immunostaining confirmed that the areas that remained white after Evans blue administration have typically a reticulated pattern, similar to normal vessel segments without stentgraft. The reticulated staining was generally seen on most areas, except at the areas corresponding the intense Evans blue stain or areas with aggregated red blood cells or thrombosis.

Histolopathological examination from transferse vessel sections was performed to confirm the findings at planimetry and SEM regarding the presence of the endothelium.

The results are summarised in the following table.

| **Animal ID, treatment, end-point** | **Planimetry ( from segments A and B)** | **SEM (segment A)** | **Histology (segment B)** |
|---|---|---|---|
| OD153, 600µg VEGF, one week | 57.9 % | 32.7 % | endothelial cells overlying media, moderate amount luminal endothelial cells |
| OD155, 600µg VEGF + 300 µg FGF-2, one week | 57.6 % | 38.9 % | endothelial cells on the mural surface of the graft memebrane, moderate amount luminal endothelial cells |
| OD156, placebo, one week | 32.9 % | 21.6 % | Luminal thrombus. A single endothelial cell on luminal graft surface observed |
| OD178, 600µg VEGF, two weeks | 55.8 % | 73.4 % | Endothelial lining almost complete. |
| OD179, placebo, two weeks | 0 %, thrombosed | 0%, thrombosed | Thrombotic occlusion |
| OD184, 600µg VEGF, two weeks | 62.0 % | 66.5 % | Luminal side mostly endothelialized. |

### Example 15.

### ePTFE stentgrafts in rabbits

This example demonstrates that binding of VEGF plasmid to a ePTFE stentgraft increases endothelialization of luminal surface.

### METHODS

Two New Zeland White rabbits (2.5-3.2 kg) were used in the experiments and underwent a bilateral stentgraft placement in the carotid arteries to study the endothelialization.

### Genetic methods:

The same genetic methods were used as described before.

### Modification of stentgraft membrane surface.

The surface was modified at Corline Systems AB, Sweden by introducing a cationic surface coating on the PTFE membrane facing the vessel wall. The plasmid solution (600 µg VEGF in approx. 50 µl sterile water) or placebo (50 µl sterile water) was applied with pipette in 5µl increments on the stentgrafl surface and air-dried until all visible water was evaporated. Immediately thereafter the stentgraft was deployed as described below. In these experiments both the left and the right carotid artery of each individual animal received identical treatment.

### Construction of stentgraft.

The stentgrafts were constructed as described previously, except for stents from another manufacturer was used (PURA-A stent, 7 mm long, 3-5 mm post-dilatation diameter, Daevon Medical, Hamburg, Germany

### Carotid artery angioplasty with insertion of stentgrafts.

The stentgrafts were implanted as described in previous example, except for the plasmid solution was applied on the graft surface as above.

### Ex vivo animal examination.

Animal sacrifice procedure was as in previous example. The carotid arteries from animals with one week end-point were explanted and the stented vessel segments were processed to methylmethacrylate (MMA) inbeddning and further histological examination.

### Histological examination.

Histological examination was performed as described in previous example.

### RESULTS

### Endothelialisation

Histolopathological examination from transferse vessel sections was performed to detect the presence of the endothelial cells.

The results are summarised in the following table.

| **Animal ID, treatment, end-point** | **Histology (complete stentgraft), dx** | **Histology (complete stentgraft), sin** |
|---|---|---|
| OD182, 600µg VEGF, one week | Few luminal endothelial cells | Luminal endothelial cells |
| OD191, control, one week | Fresh occluding thrombus | Luminal thrombus formation, single endothelial cells? |

## Claims

1. A medical device with improved biological properties for an at least partial contact with blood, bodily fluids and/or tissues when implanted in a mammalian body, which device comprises a porous synthetic core and a nucleic acid present in a biologically compatible medium, **characterised in that** said nucleic acid encodes a translation or transcription product capable of inducing *in vivo* capillary endothelialisation at least partially on at least one synthetic surface of said core.

2. A device according to claim 1, wherein said nucleic acid is at least temporarily attached to said device and is released from said device upon introduction in said mammalian body.

3. A device according to any of the claims 1-2, wherein the nucleic acid is present in the biologically compatible medium in naked form.

4. A device according to any of the claims 1-3, wherein the nucleic acid has been introduced in a viral vector selected from the group consisting of retrovirus, Sendal virus, adeno associated virus and adenovirus.

5. A device according to any of the claims 1-3, wherein the nucleic acid is present In a liposome.

6. A device according to any one of the preceding claims, wherein the nucleic acid is encoding a protein or polypeptide selected from the group consisting of fibroblast growth factor (FGF), platelet derived growth factor (PDGF), transforming growth factor (TGF) and epidermal growth factor (EGF) families, placenta derived growth factor (PIGF), hepatocyte growth factor (HGF) and angiopoetin.

7. A device according to any of the preceding claims, wherein the nucleic acid is encoding vascular endothelial growth factor (VEGF), acidic fibroblast growth factor (aFGF), basic flbrobiast growth factor (bFGF) or fibroblast growth factor-5 (FGF-5).

8. A device according to any of the preceding claims, wherein the biologically compatible medium is a biostable polymer, a bioabsorbale polymer, a biomolecule, a hydrogel polymer or fibrin.

9. A device according to any of the preceding claims, which comprises the nucleic acid in a reservoir separate from said core enabling a successive delivery thereof to a mammalian body.

10. A device according to any one of claims 1-9, wherein the nucleic acid has been attached to the core by ionic or covalent bonding.

11. A device according to any one of the preceding claims, wherein the synthetic surface is nonporous.

12. A device according to any one of claims 1-11, wherein the synthetic surface is porous and allows capillary and endothellal cell growth through pores.

13. A device according to any one of the preceding claims, which is a cardiovascular implant.

14. A device according to any one of the preceding claims, which is an endovascular implant.

15. A device according to any one of the preceding claims, which is an implant for the replacement of a part of a mammalian body.

16. A device according to the any of the claims1-15, which is a porous stent graft.

17. A device according to any of the claims 1-14, which is a porous vascular graft.

18. A device according to any of the claims 1-14 which is a porous graft connector.

19. A device according to any one of claims 1-13, which is a tissue Implant.

20. A device according to any one of claims 1-13, which is a biosensor.

21. A method for producing a medical device with improved biological properties for an at least partial contact with blood, bodily fluids and/or tissues when implanted in a mammalian body, which device comprises a porous synthetic core and a nucleic acid present in a biologically compatible medium, which comprises providing a core comprising at least one surface of a synthetic material; and providing a nucleic acid in a biologically compatible medium, **characterised In that** said nucleic acid encodes a translation or transcription product capable of inducing *in vivo* capillary endothelialisation at least partially on at least one synthetic surface of said core.

22. A method according to claim 21, wherein the nucleic acid is attached to the core by ionic or covalent bonds.

23. A method according to claim 22, wherein the nucleic acid is provided in a reservoir separate from the core to enable addition thereof at least once to the surroundings of the core after introduction Into a mammalian body.

24. Use of a nucleic acid encoding an angiogenic factor for improving the biological properties of a synthetic surface of a medical device for an at least partial contact with blood, bodily fluids and/or tissues when implanted in a mammalian body, which device comprises a porous synthetic core and a nucleic acid present in a biologically compatible medium, and wherein said nucleic acid, encoding a translation or transcription product capable of inducing *in vivo* capillary endothelialisation at least partially on at least one synthetic surface of said core, is contacted with said surface in solution or gel form.

## Patentansprüche

1. Medizinische Vorrichtung mit verbesserten biologischen Eigenschaften für einen mindestens teilweisen Kontakt mit Blut, Körperflüssigkeiten und/oder Geweben, wenn sie in einen Säugetierkörper implantiert wird, wobei die Vorrichtung einen porösen synthetischen Kern und eine Nukleinsäure, die in einem biologisch kompatiblen Medium vorliegt, umfasst,
**dadurch gekennzeichnet,**
**dass** die Nukleinsäure für ein Translations- oder Transkriptionsprodukt codiert, das fähig ist, in vivo eine Kapillarendothelialisation mindestens teilweise an mindestens einer synthetischen Oberfläche des Kerns zu induzieren.

2. Vorrichtung nach Anspruch 1, wobei die Nukleinsäure mindestens zeitweilig an die Vorrichtung gebunden ist und nach Einführung in den Säugetierkörper aus der Vorrichtung freigesetzt wird.

3. Vorrichtung nach Anspruch 1 oder Anspruch 2, wobei die Nukleinsäure in dem biologisch kompatiblen Medium in nackter Form vorliegt.

4. Vorrichtung nach einem der Ansprüche 1 bis 3, wobei die Nukleinsäure in einen viralen Vektor, ausgewählt aus der Gruppe bestehend aus Retrovirus, Sendalvirus, adenoassoziiertem Virus und Adenovirus, eingeführt worden ist.

5. Vorrichtung nach einem der Ansprüche 1 bis 3, wobei die Nukleinsäure in einem Liposom vorliegt.

6. Vorrichtung nach einem der vorangehenden Ansprüche, wobei die Nukleinsäure für ein Protein oder ein Polypeptid codiert, das aus der Gruppe bestehend aus Fibroblastenwachstumsfaktor (FGF), von Thrombozyten abgeleitetem Wachstumsfaktor (PDGF), transformierendem Wachstumsfaktor (TGF) und den Familien des epidermen Wachstumsfaktors (EGF), aus Plazenta stammendem Wachstumsfaktor (PIGF), Hepatocytenwachstumsfaktor (HGF) und Angiopoetin, ausgewählt ist.

7. Vorrichtung nach einem der vorangehenden Ansprüche, wobei die Nukleinsäure vaskulären endothelialen Wachstumsfaktor (VEGF), sauren Fibroblastenwachstumsfaktor (aFGF), basischen Fibroblastenwachstumsfaktor (bFGF) oder Fibroblastenwachstumsfaktor-5 (FGF-5) codiert.

8. Vorrichtung nach einem der vorangehenden Ansprüche, wobei das biologisch kompatible Medium ein biologisch stabiles Polymer, ein biologisch absorbierbares Polymer, ein Biomolekül, ein Hydrogelpolymer oder Fibrin ist.

9. Vorrichtung nach einem der vorangehenden Ansprüche, die die Nukleinsäure in einem Reservoir getrennt von dem Kern umfasst, was eine sukzessive Abgabe derselben an einen Säugetierkörper ermöglicht.

10. Vorrichtung nach einem der Ansprüche 1 bis 9, wobei die Nukleinsäure durch ionische oder kovalente Bindung an den Kern gebunden worden ist.

11. Vorrichtung nach einem der vorangehenden Ansprüche, wobei die synthetische Oberfläche nicht porös ist.

12. Vorrichtung nach einem der Ansprüche 1 bis 11, wobei die synthetische Oberfläche porös ist und ein Kapillar- und Endothelzell-Wachstum durch Poren erlaubt.

13. Vorrichtung nach einem der vorangehenden Ansprüche, die ein kardiovaskuläres Implantat ist.

14. Vorrichtung nach einem der vorangehenden Ansprüche, die ein endovaskuläres Implantat ist.

15. Vorrichtung nach einem der vorangehenden Ansprüche, die ein Implantat für den Ersatz eines Teils eines Säugetierkörpers ist.

16. Vorrichtung nach einem der Ansprüche 1 bis 15, die ein poröses Stent-Transplantat ist.

17. Vorrichtung nach einem der Ansprüche 1 bis 14, die ein poröses vaskuläres Transplantat ist.

18. Vorrichtung nach einem der Ansprüche 1 bis 14, die ein poröser Transplantatadapter ist.

19. Vorrichtung nach einem der Ansprüche 1 bis 13, die ein Gewebeimplantat ist.

20. Vorrichtung nach einem der Ansprüche 1 bis 13, die ein Biosensor ist.

21. Verfahren zur Herstellung einer medizinischen Vorrichtung mit verbesserten biologischen Eigenschaften für einen mindestens teilweisen Kontakt mit Blut, Körperflüssigkeiten und/oder Geweben, wenn sie in einen Säugetierkörper implantiert wird, wobei die Vorrichtung einen porösen synthetischen Kern und eine Nukleinsäure, die in einem biologisch kompatiblen Medium vorliegt, umfasst, umfassend Bereitstellen eines Kerns, der mindestens eine Oberfläche aus einem synthetischen Material umfasst, und Bereitstellen einer Nukleinsäure in einem biologisch kompatiblen Medium,
**dadurch gekennzeichnet,**
**dass** die Nukleinsäure für ein Translations- oder Transkriptionsprodukt codiert, das fähig ist, in vivo eine Kapillarendothelialisation mindestens teilweise an mindestens einer synthetischen Oberfläche des Kerns zu induzieren.

22. Verfahren nach Anspruch 21, wobei die Nukleinsäure durch ionische oder kovalente Bindungen an den Kern gebunden ist.

23. Verfahren nach Anspruch 22, wobei die Nukleinsäure in einem Reservoir getrennt vom Kern angeordnet ist, um einen Zusatz derselben mindestens einmal an die Umgebung des Kerns nach Einführung in einen Säugetierkörper zu ermöglichen.

24. Verwendung einer Nukleinsäure, die für einen angiogenetischen Faktor codiert, zur Verbesserung der biologischen Eigenschaften einer synthetischen Oberfläche einer medizinischen Vorrichtung für einen mindestens teilweisen Kontakt mit Blut, Körperflüssigkeiten und/oder Geweben, wenn sie in einen Säugetierkörper implantiert wird, wobei die Vorrichtung einen porösen synthetischen Kern und eine Nukleinsäure, die in einem biologisch kompatiblen Medium vorliegt, umfasst, und wobei die Nukleinsäure, die für ein Translations- oder Transkriptionsprodukt codiert, das fähig ist, in vivo eine Kapillarendothelialisation mindestens teilweise an mindestens einer synthetischen Oberfläche des Kerns zu induzieren, mit der Oberfläche in Form einer Lösung oder eines Gels in Kontakt gebracht wird.

## Revendications

1. Dispositif médical ayant des propriétés biologiques améliorées pour un contact au moins partiel avec du sang, des fluides et/ou des tissus corporels lorsqu'implanté dans un corps de mammifère, lequel dispositif comporte un noyau synthétique poreux et un acide nucléique présent dans un milieu biologiquement compatible, **caractérisé en ce que** ledit acide nucléique code pour un produit de traduction ou de transcription capable d'induire une endothélialisation capillaire *in vivo* au moins partiellement sur au moins une surface synthétique dudit noyau.

2. Dispositif selon la revendication 1, dans lequel ledit acide nucléique est au moins temporairement fixé audit dispositif et est libéré dudit dispositif lors de l'introduction dans ledit corps de mammifère.

3. Dispositif selon l'une quelconque des revendications 1 et 2, dans lequel l'acide nucléique est présent dans le milieu biologiquement compatible sous une forme nue.

4. Dispositif selon l'une quelconque des revendications 1 à 3, dans lequel l'acide nucléique a été introduit dans un vecteur viral sélectionné parmi le groupe constitué du rétrovirus, virus Sendai, virus adéno-associé et adénovirus.

5. Dispositif selon l'une quelconque des revendications 1 à 3, dans lequel l'acide nucléique est présent dans un liposome.

6. Dispositif selon l'une quelconque des revendications précédentes, dans lequel l'acide nucléique code pour une protéine ou un polypeptide sélectionné parmi le groupe constitué des familles du facteur de croissance de fibroblastes (FGF), du facteur de croissance dérivé de plaquettes (PDGF), du facteur de croissance transformant (TGF) et du facteur de croissance épidermique (EGF), du facteur de croissance dérivé du placenta (PIGF), du facteur de croissance d'hépatocytes (HGF) et d'angiopoïétine.

7. Dispositif selon l'une quelconque des revendications précédentes, dans lequel l'acide nucléique code pour le facteur de croissance de l'endothélium vasculaire (VEGF), le facteur de croissance de fibroblastes acide (aFGF), le facteur de croissance de fibroblastes basique (bFGF), ou le facteur de croissance de fibroblastes 5 (FGF-5).

8. Dispositif selon l'une quelconque des revendications précédentes, dans lequel le milieu biologiquement compatible est un polymère biostable, un polymère bioabsorbable, une biomolécule, un polymère hydrogel ou une fibrine.

9. Dispositif selon l'une quelconque des revendications précédentes, qui comporte l'acide nucléique dans un réservoir séparé dudit noyau permettant une libération successive de celui-ci dans un corps de mammifère.

10. Dispositif selon l'une quelconque des revendications 1 à 9, dans lequel l'acide nucléique a été fixé au noyau par liaison ionique ou covalente.

11. Dispositif selon l'une quelconque des revendications précédentes, dans lequel la surface synthétique est non-poreuse.

12. Dispositif selon l'une quelconque des revendications 1 à 11, dans lequel la surface synthétique est poreuse et permet une croissance de cellules endothéliales et capillaires à travers les pores.

13. Dispositif selon l'une quelconque des revendications précédentes, qui est un implant cardio-vasculaire.

14. Dispositif selon l'une quelconque des revendications précédentes, qui est un implant endo-vasculaire.

15. Dispositif selon l'une quelconque des revendications précédentes, qui est un implant pour le remplacement d'une partie d'un corps de mammifère.

16. Dispositif selon l'une quelconque des revendications 1 à 15, qui est une greffe stent poreuse.

17. Dispositif selon l'une quelconque des revendications 1 à 14, qui et une greffe vasculaire poreuse.

18. Dispositif selon l'une quelconque des revendications 1 à 14, qui est un connecteur de greffe poreux.

19. Dispositif selon l'une quelconque des revendications 1 à 13, qui est un implant tissulaire.

20. Dispositif selon l'une quelconque des revendications 1 à 13, qui est un biocapteur.

21. Procédé pour produire un dispositif médical ayant des propriétés biologiques améliorées pour un contact au moins partiel avec du sang, des fluides et/ou tissus corporels lorsqu'implanté dans un corps de mammifère, lequel dispositif comporte un noyau synthétique poreux et un acide nucléique présent dans un milieu biologiquement compatible, qui comporte la fourniture d'un noyau comportant au moins une surface d'un matériau synthétique, et la fourniture d'un acide nucléique dans un milieu biologiquement compatible, **caractérisé en ce que** ledit acide nucléique code pour un produit de traduction ou de transcription capable d'induire une endothélialisation capillaire *in vivo* au moins partiellement sur au moins une surface synthétique dudit noyau.

22. Procédé selon la revendication 21, dans lequel l'acide nucléique est fixé au noyau par des liaison ioniques ou covalentes.

23. Procédé selon la revendication 22, dans lequel l'acide nucléique est agencé dans un réservoir séparé du noyau pour permettre l'ajout de celui-ci au moins une fois au voisinage du noyau après introduction dans un corps de mammifère.

24. Utilisation d'un acide nucléique codant pour un facteur angiogénique destiné à améliorer les propriétés biologiques d'une surface synthétique d'un dispositif médical pour un contact au moins partiel avec du sang, des fluides et/ou tissus corporels lorsqu'implanté dans un corps de mammifère, lequel dispositif comporte un noyau synthétique poreux et un acide nucléique présent dans un milieu biologiquement compatible, et dans lequel ledit acide nucléique, codant pour un produit de traduction ou de transcription capable d'induire une endothélialisation capillaire *in vivo* au moins partiellement sur au moins une surface synthétique dudit noyau, est mis en contact avec ladite surface sous forme d'une solution ou d'un gel.
